# EUROPEAN PATENT SPECIFICATION

(11) **EP 2 284 539 B1**
(45) Date of publication and mention of the grant of the patent: **09.04.2014**
(21) Application number: 10192255.7
(22) Date of filing: 19.12.2006
(51) Int. Cl.: B82Y 40/00, G01N 33/543, B82Y 30/00, B82Y 15/00

(54) **Method and apparatus for analyzing bioprocess fluids**
Verfahren und Gerät zur Untersuchung von Bioprozess-Flüssigkeiten
Méthode et appareil pour analyser les fluides bioprocédés

(30) Priority: 19.12.2005 US 751492 P; 02.05.2006 US 416789; 02.05.2006 US 416624; 02.05.2006 US 417000; 02.05.2006 US 417005; 02.05.2006 US 416997; 02.05.2006 US 416998
(43) Date of publication of application: 16.02.2011
(62) Divisional of application: 06845757.1
(73) Proprietor: Bioscale, Inc., Cambridge, Massachusetts 02139 (US)
(72) Inventor: Masters, Brett P., Belmont, CA 94002 (US)
(74) Representative: Viering, Jentschura & Partner Patent- und Rechtsanwälte

(56) References cited:
- WO-A-2006/119308
- WO-A2-03/048998
- WO-A2-2004/053105
- US-A1- 2003 154 031
- US-A1- 2006 286 685

## Description

### TECHNICAL FIELD

The present invention relates to an apparatus for detecting one or more biological markers in bioprocess fluid samples.

### BACKGROUND OF THE INVENTION

Biological therapeutics such as proteins are produced in various animal and host cell models. Typically, the product molecule of interest is isolated from the host, host cell, or conditioned medium used to grow the host cell and is purified to remove host proteins and other contaminants before being administered to humans. Sensitive measurement of such host proteins and contaminants is desirable at various points during purification of the product molecule. Currently, techniques for detecting or measuring the host cell proteins and contaminants during purification of product molecules typically involve Enzymes Linked Immunosorbant Assay using antibodies that recognized the host cell protein or contaminant. Such assays typically take at least 5 hours to complete and are typically performed off line, that is samples are removed from the production line and taken to a separate laboratory where the assay is performed. The assays often have a detection limit of about 400 pg/ml.

Significant challenges for a system that detects analytes (e.g., biological agents or biological markers) in liquid media include concentration of the analyte in the media, and transport of the analyte to a sensor surface. For biological applications such as analysis of bioprocess fluids for host cell proteins or contaminants, concentration issues generally arise since the concentrations of such analytes tend to be low. Additionally, biological analytes (e.g., cells, cell fragments and macromolecules such as proteins and nucleic acids) tend to be relatively large; hence, transport issues arise because these larger analytes diffuse in fluid solution very slowly. In addition to cells, cell fragments, and molecules such as proteins and nucleic acids, the detection of small molecule analytes can be a useful-marker for diagnosing disease, monitoring drug pharmacokinetics in a patient, screening small molecule libraries for potential drug targets and analyzing bioprocess fluids.

There is a need for improved assays that can quickly detect low concentrations of analyte. In addition, there is a need for improved measurement of analytes including small molecule analytes. Furthermore, a need exists for highly sensitive methods and apparatus for analyzing bioprocess fluids and methods and apparatus that can be used in real time at the production line, or even in line with production in order to streamline the purification process. The ability to analyze bioprocess fluids at line or in line at various stages of the purification process is expected to allow more rapid troubleshooting of production problems, thereby minimizing production downtime.

A key metric for competitive detection is the amount of analyte accumulated on a sensor per unit time. For good performance, the rate of accumulation (and the resulting signal transient) needs to be fast relative to the sensor drift rate. Another key performance metric for an analyte detection system is the degree to which the system can preferentially collect the analyte of interest on the sensor surface. Since many biological samples contain extraneous background components (e.g., other proteins, cells, nucleic acids, dirt), it is necessary to prevent these background components from interfering with the desired measurement. So, a transport method that selectively draws the analyte to the sensor and allows interfering background components to pass by has definite advantages. Such a method used in concert with selective binding of the analyte (e.g., antibody, complimentary DNA strands, etc.) to the sensor surface can deliver high sensitivity measurements for samples with large amounts of extraneous background components relative to the amount of analyte.

Various methods for improving transport of analyte to a sensor surface have been proposed, including filtration, novel flow geometries, acoustic fields, electrical fields (time varying and static) and magnetic fields.

Acoustic excitation has been used to draw cells to field nodes, but it is difficult to use this technique alone to transport material to a surface.

Electrical fields (electrophoresis and dielectrophoresis) have been used to enhance transport but are not universally applicable to all analytes and sample types. They are generally more effective for larger analytes (e.g., cells). Furthermore, the electrical properties of microbes can vary within a given species and strain, making it hard to predict system performance under all intended operating conditions. Sometimes it is necessary to tailor the ionic strength of the sample to improve the performance of the transport. This requirement can conflict with the optimum binding or wash conditions in an assay. Also, electrical fields can dissipate energy and heat conductive fluids (e.g., 0,1 M phosphate buffer solution), which is undesirable since heating can damage the biological analytes.

Immunomagnetic separation (IMS) methods are known in the art for isolating analyte from a sample.

WO 03/048998 discloses healthcare networks with biosensors, including acoustic sensors and biosensors consisting of a piezoelectric crystal device and a chemical system attached to the crystal surface, with the chemical system consisting of a polymer coating and/or chemoreceptors and being capable of selectively binding molecules and gas particles.

### SUMMARY OF THE INVENTION

Disclosed herein are apparatuses for analyzing a bioprocess fluid. In some embodiments, a bioprocess fluid and a plurality of particles coated with a plurality of capture agents capable of binding one or more biological markers are introduced into a fluid chamber. At least one surface of the fluid chamber comprises an acoustic device having a plurality of capture agents capable of binding the one or more biological markers bound thereto. Signal output by said acoustic device is monitored, thereby detecting one or more biological markers in the bioprocess fluid.

In some embodiments, the analysis includes assessing the purity of a bioprocess fluid. For example, in some embodiments, the method using said apparatus involves detecting a level of one or more biological markers in the bioprocess fluid. The method comprises introducing the bioprocess fluid and a plurality of particles coated with a plurality of capture agents capable of binding the biological marker into a fluid chamber. At least one surface of the fluid chamber comprises an acoustic device having a plurality of capture agent capable of binding the one or more biological markers bound thereto. Signal output by said acoustic device is monitored to detect the level of one or more biological markers in the sample, thereby assessing purity of a bioprocess fluid.

In some embodiments the method using said apparatus involves detecting a level of one or more biological markers in the bioprocess fluid. The bioprocess fluid is combined with a plurality of magnetic particles that comprise a plurality of capture agents having an affinity for one or more biological markers to produce at least some magnetic particles bound to at least some of the one or more biological markers. The combined fluid is directed into a fluid chamber, wherein at least one surface of the fluid chamber comprises an acoustic device having a plurality of capture agents capable of binding the one or more biological markers bound thereto. A magnetic flux is created in proximity to the flexural plate wave device to magnetically attract at least some of the plurality of bound magnetic particles to the at least one surface of the flexural plate wave device, and signal output by said acoustic device is monitored, to detect the level of one or more biological markers in the sample, thereby assessing purity of a bioprocess fluid.

The invention relates to an apparatus for assessing purity of a bioprocess fluid as defined in the appended claims. In some embodiments, the apparatus comprises a fluid chamber having at least one opening for fluid to enter; a flexural plate wave device defining at least a portion of at least one interior surface of the fluid chamber. The at least one interior surface coated with a plurality of capture agents that are capable of binding one more biological markers. The apparatus includes an monitoring device to monitor at least one signal output by the flexural plate wave device. The apparatus includes a plurality of magnetic particles coated with a plurality of capture agents that are capable of binding one or more biological markers. The apparatus can also include a source of magnetic flux to selectively attract magnetic particles to the flexural plate wave device.

In one embodiment the biological marker binds to a magnetic particle (e.g., a magnetic bead) to form an analyte-particle complex. The analyte-particle complex is transported and localized onto the surface of a sensing device by applying a gradient magnetic field. The magnetic field induces a polarization in the magnetic material of the particle that is aligned with the local magnetic field lines. The particle experiences a net force in the direction of the gradient, causing the particle to migrate toward regions of higher field strength. The magnetic field distribution is tailored to draw analyte-particle complexes from a sample flow and distribute them across the surface of the sensing device. The extraneous, background components of the sample (e.g., cells, proteins) generally have a much lower magnetic susceptibility as compared to the magnetic particles, and so the magnetic field does not significantly influence them. Hence, only a very small fraction of this background material interacts with the sensor surface.

In one embodiment, the sensing device is a flexural plate wave (FPW) device, which functions particularly well with the magnetic particles for two reasons. First, the presence of the magnetic particles on the surface of the sensing device results in an amplified FPW signal response. The larger combined size and density of the analyte-particle complex yields a larger FPW signal response than the analyte alone. Second, the surface of the sensor in the FPW device consists of a thin membrane that is typically only a few micrometers thick, which allows larger magnetic fields and field gradients to be created at the sensor surface because the field source can be positioned closer to the sample flow. This results in higher fractional capture of the analyte from the sample. With this higher capture rate and efficiency, it is possible to process larger sample volumes in shorter times than would be otherwise possible.

In another aspect, a cartridge for a resonant device system includes a first fluid chamber having at least one opening for fluid to enter, and a flexural plate wave device defining at least one interior surface of the fluid chamber. The apparatus further includes a first source of magnetic flux to selectively attract magnetic particles to the at least one interior surface of the first fluid chamber.

In another aspect, a method for detection of a biological marker (also referred to herein as an analyte) includes combining a fluid containing an analyte (also referred to herein as bioprocess fluid) with a plurality of magnetic particles that comprise a capture agent having an affinity for the analyte to produce at least some magnetic particles bound to at least some analyte. The method further includes directing the combined fluid into a first fluid chamber, wherein at least one surface of a flexural plate wave device is in fluid communication with the fluid in the first fluid chamber. The method also includes creating a first magnetic flux in proximity to the flexural plate wave device to magnetically attract at least some of the bound magnetic particles to the at least one surface of the flexural plate wave device.

In another aspect, a method for detection of an analyte includes coating at least a portion of a surface of a flexural plate wave device located in a fluid chamber with a first capture agent, and directing a fluid containing an analyte into the fluid chamber to bind some of the analyte to the capture agent located on the flexural plate wave device. The method further includes directing a fluid containing a plurality of magnetic particles that comprise a second capture agent into the fluid chamber, and creating a magnetic flux in proximity to the flexural plate wave device to attract at least some of the magnetic particles towards the surface of the flexural plate wave device.

The methods and apparatus provided herein can be used at any stage during the processing and/or purification of the product molecule of interest. Typically, processing and/or purification of product molecules involves the stages of feed preparation, capture of the product molecule, intermediate purification and polishing. A sample of the bioprocess fluid can be tested using the method and or apparatus provided herein before, during, or after any one of the stages. The bioprocess fluid can be tested for the presence of one or more biological markers. The bioprocess fluid can also be tested for the presence and/or amount of the product molecule itself. In addition, the flow through liquid of any relevant purification step can be tested for the presence and/or amount of product molecule. In this manner, the purity of the bioprocess fluid and the yield of the product molecule of interest can be monitored.

For example, in many bioprocessing applications, the initial sample or feed stream often has properties that interfere with or reduce the efficacy of downstream purification or processing methods. The sample or feed stream may have, for example, biological markers such as nucleic acids, endotoxins, cell debris, and/or particulates. Techniques such as filtration can be used to remove the biological markers while retaining the product molecule of interest in the bioprocessing fluid. Depending on the size and concentration of the biological marker and product molecule of interest, micro-filtration, ultra filtration, or diafiltration or combinations thereof can be used. The resulting bioprocess fluid can be analyzed for the presence and/or amount of the biological markers as well as for the presence and/or amount of the product molecule.

After conditioning the initial sample, the product molecule is recovered and/or stabilized from the relatively crude feedstock. The recovery process typically involves some form of binding process, where the product molecule is reversibly bound to a solid support, while the rest of the feedstock is washed away. Reversible binding of the product molecule can be achieved, for example, using one or more antibodies that specifically bind the product molecule, ion exchange chromatography (IEC), or hydrophobic interactions. Typically biological markers that have very different pI, hydrophobicity, size, and/or antigenic properties from the product molecule are removed. Further, any biological markers that can damage the product molecule, such as proteases are also removed, while recovering a maximal amount of the product molecule. The resulting bioprocess fluid can be analyzed for the presence and/or level of the biological markers as well as the presence and/or level of the product molecule. In addition, the flow through liquid from the capture stage can be tested for the presence and/or level of product molecule that was not captured, thereby allowing the operator to optimize the efficiency of the capture process at line or in line with the process.

After the capture stage, intermediate purification can be performed. After intermediate purification, steps can be taken to remove any remaining trace contaminants or biological markers that have been difficult to remove due to the similarities with the product molecule can be removed from the bioprocess fluid. Techniques such as IEC, hydrophobic interaction chromatography, and/or gel filtration are often used for intermediate purification and polishing with more selective washing or elution buffers in order to remove biological markers that are more similar in size, charge, and/or hydrophobicity to the product molecule of interest. Like the capture stage, the flow through liquid and resulting bioprocess fluid can be analyzed for the presence and/or amount of product molecule as well as the presence and/or amount of biological markers.

As a result of the methods and apparatus described herein, it is now possible to analyze a bioprocess fluid for the presence and/or amount of product molecule as well as the presence and/or amount of biological markers such as contaminiants on the same platform. For example, one surface of the apparatus can be coated with a capture agent that binds the product molecule and another surface coated with a capture agent that binds one or more biological markers. The sample can be introduced to the first and second surfaces and the presence and/or amount of product molecule can be measured by monitoring the frequency shift of the first surface. The sample can be introduced to the second surface in conjuction with particles (also referred to herein as beads), that have been coated with capture agent as described supra. The presence and/or amount of the biological marker can be measuered using the second surface.

Where the product molecule is present at concentrations of 1 mg/ml or more, the sample of bioprocess fluid can be diluted as necessary before introducing the sample to the functionalized surface. In some embodiments, the bioprocess fluid is diluted by a factor of about 10. In other embodiments, the bioprocess fluid is diluted by a factor of about 100.

In some embodiments, the functionalized surface can be calibrated using known concentrations of analytes. In some embodiments, the functionalized surface can be reused. After use, analyte and/or analyte bound to particles can be removed by washing the surface with a suitable washing solution. In some embodiments, the washing solution can be phosphoric acid. The washing solution can be, for example, 0.1M phosphoric acid. The washing solution can be flowed over the surface for a suitable period of time to remove the analyte and/or analyte/particle complexes. In some embodiments, the washing solution is flowed over the surface for 1 minute. In this manner, the functionalized surfaces can be reused multiple times. In some embodiments, the surfaces can be reused 10 or more times. A calibrated functionalized surface need not be recalibrated after each use or after stripping away the analyte or analyte/particle complexes.

As a result of the present invention, lower levels of analyte can be detected compared to conventional assays. The methods and apparatus provided herein allow for analysis of the bioprocess fluid in less time and can be performed at the production line.

Surprisingly, the use of fewer particles per sample allows for more sensitive detection of analyte. This was found where the size of the analyte is on the order of the size of the particle, and where the analyte is a small molecule. Furthermore, devices fabricated and methods performed according to the principles of the present invention are particularly useful for capturing low concentrations of particles because the sensor surface is thin (for example, in one embodiment, on the order of several microns). In one embodiment, the thin surface is used in conjunction with a magnetic field gradient and magnetic particles. Because the sensor surface is thin, a large magnetic field gradient can be induced one the side of the sensor surface. The large magnetic field gradient enhances the attraction of the magnetic particles to the sensor surface by focusing the field close to the surface of the sensor, while allowing other material contained in the sample to flow by. In this way, lower numbers of particles can be used, thereby improving the detection limit of the system, because the magnetic field gradient serves to concentrate the particles near the sensing surface where they can interact with the bound analyte or capture agent, depending on the assay format. The magnetic field gradient is removed to allow any non specifically bound particles to be washed away. Thus, the present invention improves the accuracy and the sensitivity of analyte detection.

### BRIEF DESCRIPTION OF DRAWINGS

The foregoing and other objects of this invention, the various features thereof, as well as the invention itself, may be more fully understood from the following description, when read together with the accompanying drawings in which:

FIG. 1A shows one embodiment of an analyte detection system, as described herein.

FIG. 1B shows another embodiment of a portion of the analyte detection system shown in FIG. 1A.

FIG. 2 shows a more detailed view of the FPW sensor shown in FIG. 1A.

FIG. 3 shows a general detection protocol for using an FPW sensor in combination with analyte-particle complexes for detection of biological analytes.

FIG. 4 shows the change in the signal from multiple FPW sensors as a function of time for an exemplary detection protocol.

FIG. 5 is a summary plot showing the final signal change detected as a function of original analyte concentration.

FIG. 6 shows a time evolution plot for a detection protocol, similar to the plot shown in FIG. 4, but rather for a PSA analyte.

FIG. 7 is a schematic of two formats for attaching a capture agent to the sensing surface of the acoustic device.

FIG. 8 is a schematic of one embodiment described herein, where competitor molecule (*e.g.*, analyte) is bound to the sensing surface.

FIG. 9 is a schematic of one embodiment described herein, where the competitor molecule is the analyte of interest linked to a tag and the sensing surface of the acoustic device is coated with a capture agent that is capable of binding to the tag.

FIG. 10 is a schematic of one embodiment described herein, where the competitor molecule comprises a carrier and two or more analyte molecules of interest bound thereto, and the sensing surface of the acoustic device is coated with a capture agent that is capable of binding to the analyte of interest.

FIG. 11 shows the change in signal from multiple FPW sensors as a function of PSA concentration.

FIG. 12 shows the change in signal from multiple FPW sensors as a function of estradiol concentration in serum.

FIG. 13 is a dose response curve of FK-506 in buffer.

FIG. 14 shows the change in signal from multiple FPW sensors as a function of FK-506 concentration in buffer.

FIG. 15 is a dose response curve of FK-506 in serum.

FIG. 16 shows the change in signal from multiple FPW sensors as a function of FK-506 concentration in serum.

FIG. 17 shows the change in signal from multiple FPW sensors as a function of particle concentration.

FIG. 18 shows a dose response curve of c-Troponin-I in buffer.

FIG. 19 shows a dose response curve of c-Troponin-I in serum.

FIG. 20 shows a dose response curve of c-Troponin-I in lysed whole blood.

FIG. 21 shows a dose response curve of CHO-HSP in buffer.

### DESCRIPTION OF THE PREFERRED EMBODIMENTS

Disclosed in the context of the present invention are apparatuses for detecting one or more biological markers (also referred to herein as an analyte) in a sample, such as a bioprocess fluid, using an acoustic device. In general terms, an analyte is detected based on the ability of particles coated with a capture agent (also referred to herein as a first capture agent) to alter the resonating frequency of an acoustic device in the presence of the analyte. The capture agent is capable of binding to the analyte. A plurality of particles coated with a capture agent is exposed to the sensing surface of the acoustic device in the presence of the sample, and optionally in the presence of a competitor molecule. Depending on the type of analyte to be detected, the sensing surface is coated with a capture agent (also referred to herein as a second capture agent) capable of binding the analyte (*e.g.*, a sandwich binding format), or the sensing surface is coated with a competitor molecule (*e.g.*, a small molecule format). The sensing surface is part of a fluid chamber or channel as described herein. In addition, the coated particles and/or sample can be exposed to the sensing surface in a static mode, for example, the coated particles and/or sample can be introduced into the chamber and incubated with the period for a given period of time. In another embodiment, the coated particles can be exposed to a sensing surface in a non-static mode, for example, by flowing the coated particles through the fluid chamber or channel.

Generally, in the sandwich assay formats described herein, the amount of particles capable of binding to the sensing surface of the acoustic device is proportional to the amount of analyte present in the sample. Higher levels of analyte in the sample results in more of the capture agent on the particles being occupied by analyte from the sample. Analyte coated particles are then able to bind to the capture agent coated sensing surface. In a preferred embodiment, the particles are magnetic, and a magnetic flux is applied in proximity to the acoustic device to attract at least one of the plurality of magnetic particles toward the sensing surface.

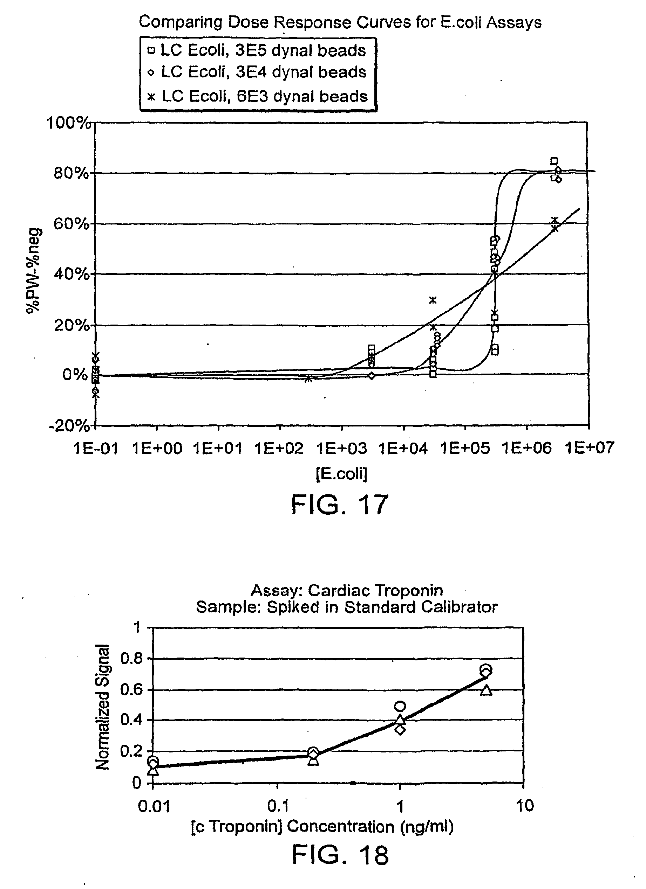

As described herein, it was surprisingly found that using a lower concentration of particles allowed for the detection of lower concentrations of analytes. The finding was unexpected because conventional wisdom in assays involving capture by particles calls for the use of a high quantity of particles in order to maximize capture efficiency. However, as shown in FIG. 17, it was found that detection limits were greatly affected by particle concentration and that use of lower particle concentrations allowed for the detection of analytes at lower concentration. Briefly, various concentrations of live *E. coli* were incubated with 3 x 10⁵, 3 x 10^{4,} or 6 x 10³ dynal beads and analyzed by the method disclosed herein.

Lower particle concentration also lowers capture efficiency. However, on balance, the large improvement in detection limit outweighed the smaller effect on capture efficiency. While not wishing to be bound by theory, in the case of an analyte of a size on the order of the particle, the binding of one analyte per particle is thought to be enough to allow the analyte-bound particle to bind the capture agent coated surface, in part because the bound analyte typically contains multiple sites that can be bound by the capture agent. Use of a large number or high concentration of particles results in many particles that have no analyte bound, which then increases the background signal, by increasing the amount of particles binding the surface non-specifically. Furthermore, if the concentration of particles is high, the non-specifically bound particles may block or prevent particles that have bound analyte from binding to the surface. The particles can be used in a concentration from 1 x 10² to 1 x 10⁷ particles/mL. In another embodiment, the particles are at a concentration of 5 x 10³ to 5 x 10⁵ particles/mL.

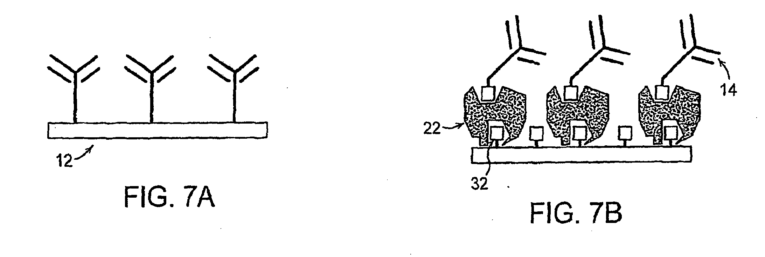

FIG. 7 shows two embodiments where the sensing surface 12 of the acoustic device is coated with a capture agent. In panel, A, the capture agent 14 is bound directly to the sensing surface 12. In panel B, the capture agent 14 is labeled with a first member of a binding pair 32. The surface is coated with the first member of a binding pair 32 and the second member of the binding pair 22.

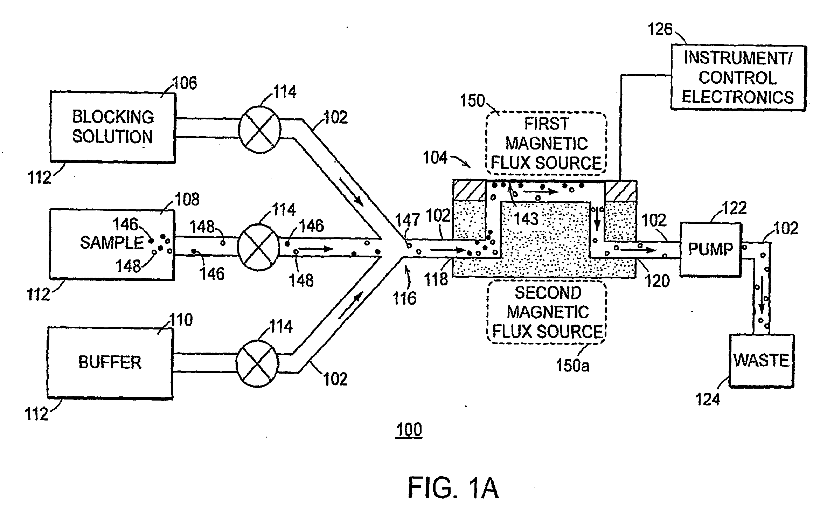

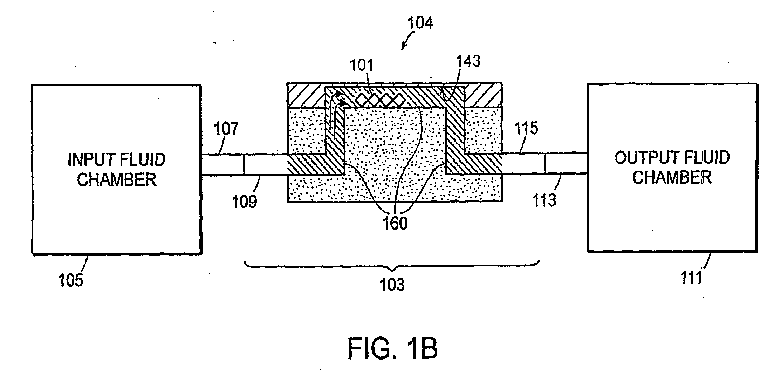

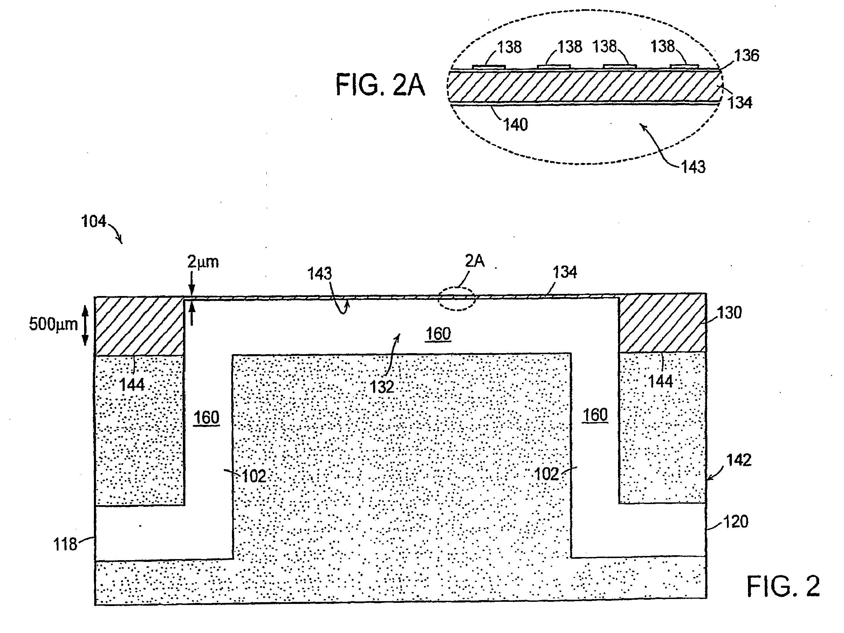

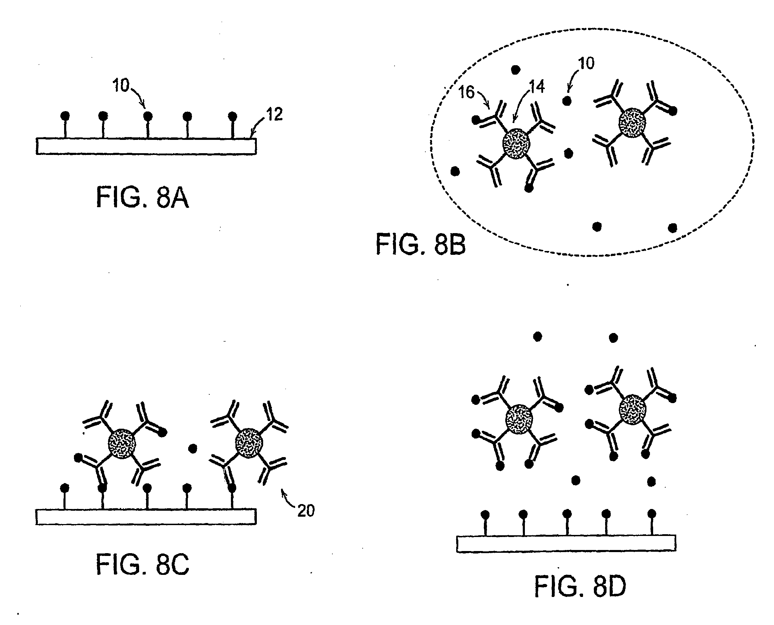

As shown in FIG. 8, in one embodiment of the small molecule format, the competitor molecule 24 comprises the analyte of interest 10 bound to the sensing surface 12 of the acoustic device (panel A). Particles 14 coated with capture agent 16 are exposed to sample containing the analyte 10 (panel B). Particle-bound capture agent that that has not bound analyte from the sample 20 is free to bind the analyte bound to the surface of the acoustic device (panel C). As shown in panels C and D, higher levels of analyte in the sample result in fewer particles binding the acoustic device sensing surface because more of the capture agent present on the particles is occupied with analyte from the sample. Signal output by the acoustic device is monitored to determine the amount and/or number of particles that have bound to the surface, thereby detecting whether one or more analytes is present in a sample. In addition, the presence or amount of analyte present in the sample can be determined, for example by comparing the signal to a control. In an alternative embodiment, as described herein, the particles can be magnetic particles. After introducing the particles into the fluid chamber, magnetic flux is created in proximity to the acoustic device to attract at least one of the plurality of magnetic particles toward the sensing surface (similarly as described, for example, in FIGS 1A, 1B and 2).

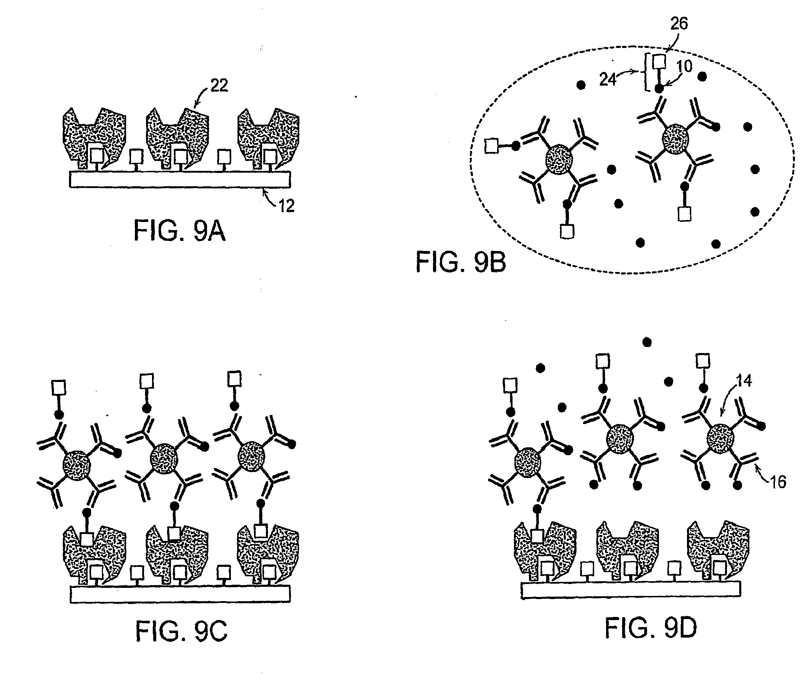

In another embodiment of the small molecule format, as shown in FIG. 9, the sensing surface 12 of the acoustic device is coated with a capture agent 22 (also referred to herein as a second capture agent) that is capable of binding to the competitor molecule 24. The competitor molecule can be, for example, the analyte of interest 10 bound to a tag 26, and the second capture agent is capable of binding to the tag. As shown in FIG. 9, higher levels of analyte in the sample results in fewer particles 14 binding to the sensing surface of the acoustic device because more of the capture agent present on the particles 16 is occupied with analyte from the sample. Because the second capture agent is capable of binding to the tag portion of the competitor molecule, the coated particles bind to the sensing surface of the acoustic device only if the particles have bound the competitor molecule.

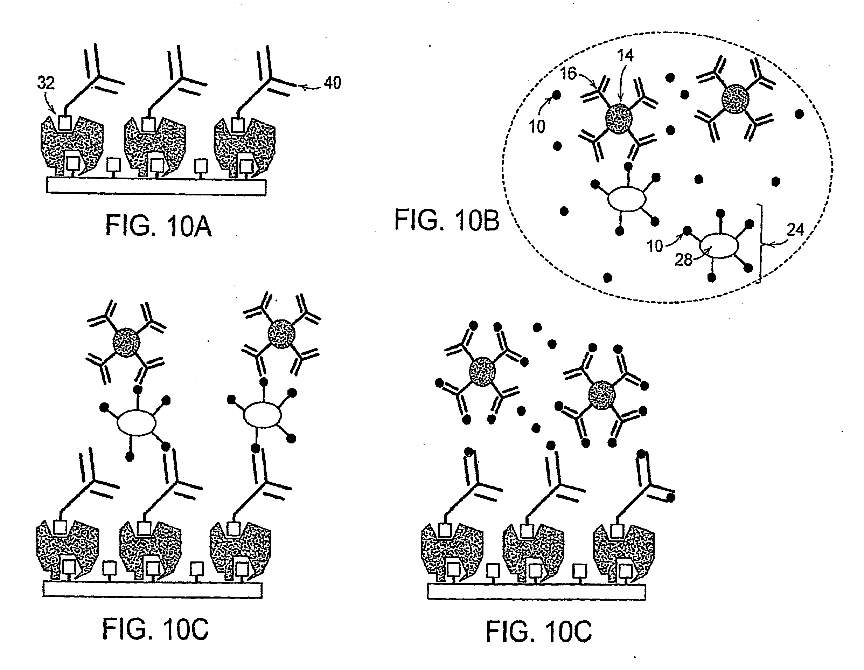

In another embodiment of the small molecule format, as shown in FIG. 10, the competitor molecule 24 can be, for example, two or more analyte molecules or moieties of interest 10 bound to a carrier 28 and the second capture agent 40 is capable of binding the analyte. As shown in FIG. 10, the second capture agent can be indirectly bound to the acoustic device sensing surface. The surface-bound and particle-bound capture agents can be the same capture agent. Particles 14 coated with capture agent 16 are exposed to sample containing the analyte 10 and the competitor molecule 24 (panel B). Particle-bound capture agent that that has not bound analyte from the sample is free to bind the competitor molecule, which in turn is bound by the capture agent that is bound to the sensing surface (panel C). As shown in panel D, higher levels of analyte present in the sample results in fewer particles binding the sensing surface of the acoustic device because more of the capture agent present on the particles is occupied with analyte from the sample. Where the analyte is a small molecule having only one copy of a given epitope or binding site, the coated particles will bind to the acoustic device sensing surface only if the particles have bound to the competitor molecule.

The method described herein can be used to determine the concentration or level of the analyte in the sample. The concentration detected or measured by the method of the present invention can be an absolute concentration or a relative concentration. For example, the concentration may be a ratio relative to the concentration of a reference analyte present in the same sample of body fluid. The concentration can be obtained by comparing the data with similar data obtained at a different time to determine whether a significant change in actual concentration has occurred. In addition, the present invention can be used in a binary format that provides a read-out that is positive if the amount of analyte detected is above a predetermined level or concentration and/or negative if the amount of analyte detected is below a predetermined level or concentration. In other embodiments, the method can provide a positive read-out if a certain threshold level of analyte is detected.

### SAMPLES

Samples suitable for use in the present invention includes any material suspected of containing the analyte. The sample can be used directly as obtained from the source or following one or more steps to modify the sample. In one embodiment, the sample is a biological sample. The sample can be derived from any biological source, such as a physiological fluids (*e.g.*, blood, saliva, sputum, plasma, serum, ocular lens fluid, cerebrospinal fluid, sweat, urine, milk, ascites fluid, synovial fluid, peritoneal fluid, amniotic fluid, and the like) and fecal matter. The sample can be obtained from biological swabs such as nasal or rectal swabs. In addition, the sample can be biopsy material. The sample can be obtained from a human, primate, animal, avian or other suitable source. In some embodiments, the sample is a bioprocess fluid. The bioprocess fluid can contain, for example, a product molecule that is to be purified from the bioprocess fluid. The bioprocess can be any sample that contains or is expected to contain the product molecule. For example, the bioprocess fluid can be conditioned tissue culture medium, milk, blood, plasma, plasma fractions, ground tissue, urine, ascites fluid and the like.

The bioprocess fluid can contain recombinantly produced product molecule. In some embodiments, the bioprocess fluid is conditioned medium produced by a recombinant host cell. As described herein, the recombinant host cell can be any suitable cell that has been altered to generate the product molecule, for example, by expressing an exogenous gene or nucleic acid that codes for the product molecule. In some embodiments, the recombinant host cell is selected from the group consisting of bacteria, fungi, insect cells, and mammalian cells into which one or more exogenous nucleic acid sequences have been added. Such nucleic acid sequences include constructs that are capable of expressing, for example, a protein of interest. The exogenous nucleic acid sequence can be introduced into the host cell by, for example, transfection using suitable transfection methods known in the art, or can be introduced by infection using a suitable viral vector, such that the protein encoded by the exogenous nucleic acid is expressed in the host cell. Suitable bacteria include, for example, *E. coli.* Suitable fungi include, for example, *Saccharomyces cerevisiae* or *Pichia pastoris.* Suitable insect cells include, for example, SF9 cells. Recombinant host cells include mammalian cells. Suitable recombinant mammalian cells include, for example, hybridoma cells or transfected mammalian cells. Suitable transfected mammalian cells include, for example, SP2/0 cells, NS/0 cells, Chinese Hamster Ovary (CHO) cells, or Human Embryonic Kidney (HEK) 293 cells to which exogenous nucleic acid has been added as described above.

In other embodiments, the bioprocess fluid can be one or more liquids selected from the group consisting of milk, conditioned cell culture media, blood, serum, plasma, and plasma fractions. In other embodiments, the bioprocess fluid comprises one or more liquids selected from the group consisting of cell homogenates and tissue homogenates.

In some embodiments, the bioprocess fluid can contain an endogenous product molecule, for example, clotting factors and immunoglobulin can be isolated from blood or fractions thereof.

As described above, the methods and apparatus provided herein can be used to analyze bioprocess fluids at all stages of the process used to purify the product from the starting material. As used herein, bioprocess fluid includes the sample as initially obtained (as conditioned cell culture medium, blood, plasma, plasma fractions, milk, tissue homogenates and the like), or can be the product of one or more processing steps to which the sample is subjected during the procedure used to isolate or purify the product from the sample. In one embodiment, the bioprocess fluid has been subjected to one or more purification steps. Such purification steps include, for example, centrifugation, filtration, diafiltration, size fractionation, affinity chromatography, IEC, HIC, gel filtration, dilution, and the like.

In some embodiments, the purified product molecule contains no more than about 1-10 ng/ml of host cell protein, such as CHO-HCP, no more than about 1 ng/ml of endotoxin, no more than about 0.1 ng/ml of DNA, no more than about 1 % of aggregates of the product molecule, and sub milligram quantities of protein A.

As described below, the sample can be pretreated prior to use, such as preparing plasma from blood, diluting viscous fluids, and the like. The sample can also be filtered, distilled, extracted, digested with enzyme or concentrated. In one embodiment, a blood sample is obtained from an individual and centrifuged and the plasma is analyzed. The sample can also be treated to inactivate or modify certain activities in the sample capable of interfering with the analyte or the detection process. For example, a decomplexing antagonist can be added to the sample to disassociate the analyte from other molecules that may be bound to and/or may interfere with the ability of the capture agent to bind to the analyte. Such antagonists can be, for example, steroid antagonists. In the case of estradiol detection, the sample can be treated by adding danazol to disassociate estradiol from sex hormone binding protein.

Other samples besides physiological fluids and solids can be used, such as water, food products, and the like, for the performance of environmental or food production assays. For example, the sample can be meat, or poultry wash (the solution used to wash poultry). In addition, a solid material suspected of containing the analyte can be used as the test sample. A solid test sample can be modified (e.g., homogenized, extracted, stomached, or solubilized) to form a liquid medium or to release the analyte.

The sample volume can be as little as 10 µl or as much as 250 ml. In another embodiment, the sample volume is about 1 to about 5 ml.

### CAPTURE AGENTS

Suitable capture agents for use in the present invention include any molecule capable of binding to one or more analytes (*e.g.*, biological markers or product molecule) of interest. The term "capture agent" includes molecules or multi-molecular complexes that can bind to an analyte or biological marker of interest. Capture agents preferably bind to their binding partners in a substantially specific manner. Capture agents with dissociation constants (KD) of less than about 10⁻⁶ are preferred. The capture agent can also be, for example, polypeptides, nucleic acids, carbohydrates, nucleoproteins, glycoproteins, glycolipids and lipoproteins.

In some embodiments, it is desireable to detect the presence or amount of product molecule in the bioprocess fluid. Capture agents includes include molecules that can bind to the product molecule. In some embodiments, the capture agent is protein A, protein G, or other molecule capable of binding to immunoglobulin.

Antibodies or antibody fragments are highly suitable as capture agents. Antigens may also serve as capture agents, since they are capable of binding antibodies. A receptor which binds a ligand is another example of a possible capture agent. Protein-capture agents are understood not to be limited to agents which only interact with their binding partners through noncovalent interactions: Capture agents may also optionally become covalently attached to the proteins which they bind. For example, the capture agent may be photocrosslinked to its binding partner following binding.

As used herein, "plurality of capture agents" includes a mixture of different capture agents such that two or more proteins or two or more antigens that comprise the one or more biological markers can be bound by the plurality of capture agents.

The term "antibody" includes any immunoglobulin, whether naturally produced or synthetically produced in whole, or in part. Derivatives of antibodies that maintain the ability of the antibody to bind to the analyte of interest are also included in the term. The term also includes any protein having a binding domain which is homologous or largely homologous to an immunoglobulin binding domain. These proteins may be derived from natural sources, produced synthetically in whole or in part. In one embodiment, the plurality of capture agents comprises a polyclonal antibody. In another embodiment, the plurality of capture agents comprises a combination of two or more monoclonal antibodies. The antibody may be a member of any immunoglobulin class, including IgG, IgM, IgA, IgD, and IgE. Where the analyte is known to bind a carrier protein, the antibody can be specific for the free form of the analyte or the carrier-bound form of the analyte. Antibodies that are capable of binding an analyte of choice can be obtained commercially or produced using known methods for generating antibodies.

The term antibody also includes antibody fragments. The term "antibody fragments" refers to any derivative of an antibody which is less than full-length. Preferably, the antibody fragment retains at least the ability to bind the analyte of interest. Examples of antibody fragments include, but are not limited to, Fab, Fab', F(ab')₂, scFv, Fv, dsFv diabody, and Fd fragments. The antibody fragment may be produced by any means. For instance, the antibody fragment may be enzymatically or chemically produced by fragmentation of an intact antibody or it may be recombinantly produced from a gene encoding the partial antibody sequence. Alternatively, the antibody fragment may be synthetically produced in whole or in part. The antibody fragment may optionally be a single chain antibody fragment. Alternatively, the fragment may comprise multiple chains which are linked together, for instance, by disulfide linkages. The fragment may also optionally be a multimolecular complex. A functional antibody fragment will typically comprise at least about 50 amino acids and more typically will comprise at least about 200 amino acids.

Single-chain Fvs (scFvs) are recombinant antibody fragments consisting of only the variable light chain (V_{L}) and variable heavy chain (V_{H}) covalently connected to one another by a polypeptide linker. Either V_{L} or V_{H} may be the NH₂ -terminal domain. The polypeptide linker may be of variable length and composition so long as the two variable domains are bridged without serious steric interference. Typically, the linkers are comprised primarily of stretches of glycine and serine residues with some glutamic acid or lysine residues interspersed for solubility. "Diabodies" are dimeric scFvs. The components of diabodies typically have shorter peptide linkers than most scFvs and they show a preference for associating as dimers. An "Fv" fragment is an antibody fragment which consists of one V_{H} and one V_{L} domain held together by noncovalent interactions. The term "dsFv" is used herein to refer to an Fv with an engineered intermolecular disulfide bond to stabilize the V_{H} -V_{L} pair. A "F(ab')₂ " fragment is an antibody fragment essentially equivalent to that obtained from immunoglobulins (typically IgG) by digestion with an enzyme pepsin at pH 4.0-4.5. The fragment may be recombinantly produced. A "Fab"' fragment is an antibody fragment essentially equivalent to that obtained by reduction of the disulfide bridge or bridges joining the two heavy chain pieces in the F(ab')₂ fragment. The Fab' fragment may be recombinantly produced. A "Fab" fragment is an antibody fragment essentially equivalent to that obtained by digestion of immunoglobulins (typically IgG) with the enzyme papain. The Fab fragment may be recombinantly produced. The heavy chain segment of the Fab fragment is the Fd piece.

Suitable polypeptide capture agents also include virtually any peptide, polypeptide, or protein that is capable of binding to an analyte of interest, or a small molecule such as a small organic molecule. In one embodiment, the capture agent is an antibody that is capable of binding to the analyte of interest. Suitable polypeptide capture agents may be obtained, for example, commercially, using recombinant methods, using synthetic production methods, or by purification from a natural source. Polypeptides include, for example, cell surface proteins, cell surface and soluble receptor proteins (such as lymphocyte cell surface receptors, steroid receptors), nuclear proteins, signal transduction molecules, transcription factors, allosteric enzyme inhibitors, clotting factors, enzymes (*e.g.*, proteases and thymidylate synthetase, serine/threonine kinases, threonine kinases, phosphatases, bacterial enzymes, fungal enzymes and viral enzymes), proteins associated with DNA and/or RNA synthesis or degradation and the like. As described in more detail below, where more than one capture agent is used, the capture agents can be, for example, isoforms of each other.

In a particular embodiment, where the analyte is a virus, the capture agent can be a cell surface receptor for the virus. For example, where the virus is HIV, the capture agent can be Dendritic cell-specific ICAM-3 grabbing nonintegrin (DC-SIGN) or CD4. In another embodiment the capture agent can be antibodies that are capable of binding viral antigens. For example, the antigen can be gp41 or gp120. The capture agent can be antibodies capable of binding host-derived antigens. For example, the antigen can be CD44, CD54, human leukocyte antigen (HLA) such as HLA-DR, or HLA-DRDPDQ.

The capture agent can also be a nucleic acid such as RNA or DNA, or peptide nucleic acid. In one embodiment, the nucleic acid or peptide nucleic acid is capable of hybridizing to nucleic acid or peptide nucleic acid analyte. In addition, the capture agent can be an aptamer, a nucleic acid capable of binding to non-nucleotide analyte (*e.g.*, proteins, small organic molecules, or inorganic molecules). As used herein, an aptamer can be either an RNA or a DNA chain composed of naturally occurring or modified nucleotides.

Suitable capture agents also include members of binding pairs. Suitable binding pairs include, for example, biotin and avidin or biotin and derivatives of avidin (*e.g.*, streptavidin and neutravidin).

Capture agents can be bound to the surface or to the bead as described below or by using standard techniques for attaching polypeptides, nucleic acids, protein A, and the like to surfaces.

### ANALYTES

As used herein, the term "analyte" refers to, for example, the molecular structure that is recognized by a capture agent. For example, the term analyte can refer to the epitope recognized by an antibody, or can include that part of a ligand that is bound by a receptor. The term analyte also includes larger molecules that contain a molecular structure that is recognized by a capture agent. The analyte can be part of a cell, for example a cell surface protein. The analyte can be an analyte of interest, chosen by the user (e.g., preselected). The analyte can be selected based on the ability to bind a capture agent of interest, for example in small molecule library screening.

In some embodiments, the analyte is a biological marker that may be present in a bioprocess fluid. As described herein, analysis of bioprocess fluids can be performed using the apparatus described herein in conjunction with a variety of commonly available commercial and specially raised capture agents, depending on the biological marker of interest. Based on the information provided in Example IX, one of ordinary skill in the art would be able to select an appropriate capture agent for the biological marker of interest, and be able to analyze the bioprocess fluid for the presence and/or amount of the biological marker. For example, capture agents that bind endotoxin and capture agents that bind protein A are commercially available.

The presence of one or more biological markers of a panel of biological markers can be assessed or detected. The panel of analytes can include one or more analytes that are detected using a competition format as described herein. The panel of analytes can include one or more analytes that are detected using the sandwich assay format as described herein. In one embodiment, each analyte is detected using a separate cartridge as described below In order to test a panel of one or more analytes, a single sample can be divided into two or more aliquots. Each aliquot can be tested for a different analyte, for example, using a different cartridge for each analyte to be tested. In this manner, panels of different analytes may be tested without requiring that multiple samples be acquired and/or that different types of apparatus be employed to test the detect the different analytes.

As used herein, the biological marker can be any component, other than the product molecule, present or suspected of being present in the starting material or introduced into the bioprocess fluid during processing. The nature of the biological marker or markers will be based in part on the source of the bioprocess fluid, that is conditioned cell culture media, blood, milk, etc. In some embodiments, the biological marker comprises host cell protein (HCP). HCP can be, for example, a mixture of proteins or antigens that are produced and/or shed by the host cell into the culture medium. In other embodiments, the biological marker can be, for example, nucleic acids, proteinases, or viruses. In other embodiments, the biological marker or markers can comprise one or more milk antigens, serum antigens, or plasma antigens from the host animal. In addition, the biological marker can be an aggregate of the product of interest, such as aggregates of immunoglobulin, variants of the product molecule, deglycosylated versions of the product molecule, and the like.

In some embodiments, the one more biological markers comprises one or more contaminants. Such contaminants can be introduced, often unintentionally, during the purification process. For example, protein A or protein G columns are often used to purify antibody from a bioprocess fluid. However, a small amount of protein A or protein G may be shed from the column, thereby contaminating the bioprocess fluid. In some embodiments, the contaminants are microbes or microbial antigens, as described, *supra,* including for example, protein A, protein, G, endotoxin, lipopolysaccharide, and other microbial toxins. In some embodiments, the one or more biological markers comprises one or more reagents that is added to the bioprocess fluid to facilitate isolation or purification of the product molecule, but that is removed in the final product.

As used herein, the one or more biological markers can be any combination of biological markers described *supra.* Product molecules can be distinguished from biological markers by a number of criteria including, for example, antigenicity, molecular weight, pI, hydrophobicity, stability, and the like.

In one embodiment, the analyte of interest is a small molecule. Small molecules include organic or inorganic molecules having a molecular weigh of about 1000 g/mol or less. Typically, small molecule analyte will contain a single or only a few binding sites. Because a small molecule has a few or only one binding site, the present invention uses competitive binding to detect and/or quantify small molecule analytes.

The small molecule can include, for example, steroids, lipids, carbohydrates, peptides, and heterocyclic compounds (e.g. bases, including co-factors such as FAD and NADH). The analyte (*e.g.*, small molecule) can be part of a library of small organic molecules which comprise aldehydes, ketones, oximes, hydrazones, semicarbazones, carbazides, primary amines, secondary amines, tertiary amines, N-substituted hydrazines, hydrazides, alcohols, ethers, thiols, thioethers, thioesters, disulfides, carboxylic acids, esters, amides, ureas, carbamates, carbonates, ketals, thioketals, acetals, thioacetals, aryl halides, aryl sulfonates, alkyl halides, alkyl sulfonates, aromatic compounds, heterocyclic compounds, anilines, alkenes, alkynes, diols, amino alcohols, oxazolidines, oxazolines, thiazolidines, thiazolines, enamines, sulfonamides, epoxides, aziridines, isocyanates, sulfonyl chlorides, diazo compounds and/or acid chlorides, preferably aldehydes, ketones, primary amines, secondary amines, alcohols, thioesters, disulfides, carboxylic acids, acetals, anilines, diols, amino alcohols and/or epoxides, most preferably aldehydes, ketones, primary amines, secondary amines and/or disulfides and combinations thereof.

The analyte of interest can also be a polypeptide, a nucleic acid, a carbohydrate, a nucleoprotein, a glycopeptide or a glycolipid. Useful analytes include, for example, enzymes, steroids, hormones, transcription factors, growth factors, immunoglobulins, steroid receptors, nuclear proteins, signal transduction components, allosteric enzyme regulators, and the like. Analytes of interest can be obtained, for example, commercially, recombinantly, synthetically, or by purification from a natural source. In preferred embodiments, the analyte of interest is associated with a specific human disease or condition.

Suitable growth factors include, for cytokines such as erythropoietin/EPO, granulocyte colony stimulating receptor, granulocyte macrophage colony stimulating receptor, thrombopoietin (TPO), IL-2, IL-3, IL-4, IL-5, IL-6, IL-10, IL-11, IL-12, growth hormone, prolactin, human placental lactogen (LPL), CNTF, and octostatin. Suitable steroids include, but are not limited to, estradiol, progesterone, testosterone, and derivatives thereof. Other suitable analytes include, for example, insulin, insulin-like growth factor 1 (IGF-1), epidermal growth factor (EGF), vascular endothelial growth factor (VEGF), placental growth factor (PLGF),TGF-α and TGF-β), other hormones and receptors such as bone morphogenic factors, follicle stimulating hormone (FSH), and leutinizing hormone (LH), tissue necrosis factor (TNF), apoptosis factor-1 and -2 (AP-1 and AP-2), and mdm2.

In one embodiment, the analyte is a cardiac marker. Cardiac markers are well known in the art and include, for example, c-troponins I and T, myoglobin, creatin kinase MB (CK-MB), and ischemia modified albumin. In one embodiment, the present invention can be used to detect a panel of analytes in order to assess a patient's condition. In one embodiment, a panel of cardiac markers is tested. The panel can include, for example, c-troponin-I, CK-MB, and myoglobin analytes.

In another embodiment, the analyte is a marker for vial infection. Markers for viral infection include, for example, inflammatory markers, circulating viral proteins, CD4+ cells, liver enzymes, and anti-virus antibodies.

The analyte of interest can also be a therapeutic drug where it would be useful to measure the levels of the drug in a patient sample, for example for drug management purposes or patient compliance. Suitable therapeutic drugs include, but are not limited to protease inhibitors and immunosupressants. Suitable protease inhibitors include ageneraser, reyataz, lexiva, telzir, crixivan, kaletra, viracep, norvi, invirase, aortovase, aptivus and the like). Suitable immunosuppressants include cyclosporin, tacrolimus (FK-506), rapamycin, mycophenolic mofetil and the like.

The analyte of interest can be a pathogen or microbe, such as bacteria or bacterial spores, viruses, parasites, prions or other pathogens or their cell wall or surface components such as gram-positive peptidoglycans, lipoteichoic and teichoicacids, and gram-negative endotoxin (e.g.) lipopolysaccharide). Bacterial analytes include, for example, Shigella sp. such as Shigella dysenteriae, Campylobacter sp. such as Campylobacter jejuni, Enterococcus sp. such as Enterococcus faecalis, Bacillus *anthracis, Yersinia pestis, Bordetella pertussis, Streptococcal* species, *Staphylococcus aureus, Mycobacterium tuberculosis, Clostridium difficile, Clostridium tetani, Clostridium botulinum, Escherichia coli, Salmonella thyphimurim, Salmonella enterica, Chlamydia species, Treponema pallidum, Neisseria gonorrhoeae, Borrelia burgdorferi, Vibrio cholerae,* Corynebacterium diphtheriae, and Helicobacter pylori. Parasites include, for example, Giardia, malaria and crytosporidia. Viral analytes include, for example, Rhinovirus, Yellow Fever, Group B Coxsachieviruses, (CB1, CB2, CB3, CB4, CB5, and CB6), Canine Parvovirus (CPV), Herpes Simplex virus type 1 (HSV1), Vaccina Virus, T4-like virus, Adenovirus, Influenza B virus, Influenza A, Avian flu, rhinovirus, coronavirus (e.g., SARS), Human Immunodeficiency virus (HIV), Hepatitis viruses, Herpes virus, West Nile Virus, and Ebola virus.

As described above, in some embodiments, more than one analyte is detected. In one embodiment, the analytes have the same molecular structure. The analytes can be, for example the same molecular species of interest. In another embodiment, the different analytes (also referred to herein as the first and second analytes) are part of a larger molecule. Therefore, the analyte can be a particular binding site (such as an epitope) attached to or contained within a larger molecule. As such, the different analytes being detected can be part of different molecules.

Analytes can be bound to the surface or to the bead as described below or by using standard techniques for attaching polypeptides, nucleic acids, and the like to surfaces. In one embodiment, the analyte is indirectly bound to the surface. The analyte can be indirectly bound to the surface, for example, by coating the surface with a first member of a binding pair. The analyte is bound or attached to a second member of the binding pair and then the analyte is bound to the surface via the interaction between the first and second members of the binding pair. Suitable binding pairs include, for example, biotin and avidin or biotin and derivatives of avidin such as streptavidin and neutravidin.

### ASSAY FORMATS

As described above, a plurality of magnetic particles is introduced into a fluid chamber. The magnetic particles are coated with a first capture agent capable of binding the analyte and at least one surface of the fluid chamber comprises an acoustic device that has been coated with a second capture agent capable of binding to the competitor molecule. The surface can be coated with the second member of the binding pair using any suitable method. For example, the surface can be coated with biotin as described below and then the biotinylated surface can be exposed to avidin or a derivative of avidin, and the second capture agent can be linked to biotin.

In another embodiment, a plurality of magnetic particles and a competitor molecule are introduced into a fluid chamber. The magnetic particles are coated with a first capture agent capable of binding the analyte. At least one surface of the fluid chamber comprises an acoustic device that has been coated with a second capture agent capable of binding to the competitor molecule. In one embodiment, the competitor molecule comprises the analyte linked or bound to a tag, and the second capture agent is capable of binding to the tag. The tag can be any moiety that can be recognized by a capture agent. In one embodiment, the tag is one member of a binding pair and the capture agent is the other member of the binding pair. For example, the tag can be biotin, and the capture agent can be avidin, streptavidin, or neutravidin. In this embodiment, the biotin is linked to the analyte using known methods for linking molecules to biotin, to form the competitor molecule. The surface can be coated with the second member of the binding pair using any suitable method. For example, the surface can be coated with biotin as described below and then the biotinylated surface can be exposed to avidin or a derivative of avidin.

In another embodiment, the competitor molecule comprises two or more analyte molecules bound to a carrier, and the second capture agent is capable of binding the analyte. The carrier can be any molecule to which two or more analyte molecules can be linked or bound. The carrier can be a protein, a nucleic acid, or other polymer. In one embodiment, the carrier is an albumin, such as bovine serum albumin. In another embodiment, the carrier is horseradish peroxidase. In this embodiment, two or more analyte molecules can be linked to the carrier as described below, or known linking technology can be used to form the competitor molecule. The surface can be coated with the capture agent as described below.

According to one embodiment of the method disclosed herein, the plurality of particles can be exposed to the sample using a variety of different orders of exposure. For example, in one embodiment, the plurality of particles is exposed to the sample and then introduced into the fluid chamber. The sample may be concentrated prior to introducing the sample-exposed particles into the fluid chamber. The sample may be concentrated by, for example, removing the particles from the solution and resuspending the particles in a smaller volume of liquid.

In another embodiment, the sample is introduced into the fluid chamber prior to introducing the plurality of particles. In still another embodiment, the plurality of particles is introduced into the fluid chamber prior to adding the sample to the fluid chamber.

In another embodiment, as described above, the plurality of particles is exposed to the sample and to a competitor molecule. The plurality of particles can be exposed to the sample and to the competitor molecule in a number of different orders. For example, in one embodiment, the plurality of particles is exposed to the sample and to the competitor molecule and then introduced into the fluid chamber. In another embodiment, the sample and the competitor molecule are introduced into the fluid chamber prior to introducing the plurality of particles. In still another embodiment, the plurality of particles is introduced into the fluid chamber prior to adding the sample and/or the competitor molecule to the fluid chamber.

### Control signal/normalization

In another embodiment, the signal output of the acoustic device in response to sample exposure is compared to or normalized with a control signal. The control signal can be provided to or obtained by the user. For example, the control signal can be a value provided to the user based on the specific analyte, capture agent, or particular model, version or type of device being used. In one embodiment, the control signal is obtained on a lot basis. For example, the control signal can be a signal that is representative of a particular lot of analyte acquired by the user. The representative signal can be, for example, experimentally derived before, during or after testing of a sample. In some embodiments, the control signal is a standard curve that is obtained by, for example, analyzing known quantities of analyte with a specific capture agent and specific version of an acoustic device.

In another embodiment, the control signal is obtained on a use basis. For example, a unique control signal can be obtained each time a particular analyte and/or capture agent is tested on a particular acoustic device. In one embodiment, the control signal is obtained using the same analyte and/or capture agent, however, in the absence of sample. The control signal can be obtained by introducing a second plurality of particles into the fluid chamber that are coated with a capture agent. At least one surface of the fluid chamber comprises an acoustic device that has a second analyte bound thereto. Signal output by said acoustic device is monitored to determine a control signal to be used in subsequent testing.

As described below for multiplexing, the different pluralities of magnetic particles can be exposed to the same or different surfaces of the fluid chamber.

### ACOUSTIC DEVICES

Acoustic devices couple to fluids predominantly through acoustic interaction between the device and the fluid. Typical acoustic devices include surface acoustic wave devices, flexural plate wave devices, lamb wave devices and cantilever devices. Acoustic devices also couple to fluids through some viscous interaction between the device and the fluid, however, the coupling is predominantly acoustic coupling. Viscous interaction devices couple to fluids predominantly through viscous interaction between the devices and the fluid. Typical viscous interaction devices include quartz microbalance (QCM) devices, shear harmonic surface acoustic wave devices, and acoustic plate mode devices. The term "surface acoustic wave" refers to the manner in which energy is carried in the device structure rather than how the device couples to the fluid. Acoustic devices are devices where fluid interacts over a substantial area of a plane of the device. Acoustic devices respond with substantial out of plane motion that couples acoustically to fluid in proximity to the plane of the device (i.e., kinetic energy, potential energy and losses are carried predominantly in the fluid). Viscous interaction devices respond primarily with in-plane motion that does not couple acoustically to fluid in proximity to a plane of the device.

For applications involving, for example, the detection and quantification of biological or chemical substances in a fluid, the coupling between an acoustic device and a fluid is typically between about 100 nm and about 10 microns in thickness relative to the plane of the device where the coupling between a viscous interaction device and a fluid is between about 10 nm and about 100 nm in thickness relative to the plane of the device.

Surface acoustic wave devices and shear harmonic surface acoustic wave devices both carry energy in their respective structures in similar manner. Surface acoustic wave devices acoustically couple significantly to fluids while shear harmonic surface acoustic wave devices couple to fluids predominantly through viscous interaction.

One embodiment of an analyte detection system 100, constructed according to the invention, is shown in FIG 1A. The system 100 includes a network of channels 102 for transporting various test solutions (also referred to herein as "test fluids" or "fluids") through an FPW device 104. The following U.S. Patents and Patent Applications describe examples of the various types of FPW devices suitable for use in the present invention: U.S. Patent No. 5,129,262, U.S. Patent No. 5,189,914, U.S. Patent No. 6,688,158 B2, U.S. Patent Application No. 10/324,685, U.S. Patent No. 5,668,303, U.S. Patent No. 5,836,203, and U.S. Patent Application 20040038195.

For example, U.S. Patent No. 5,129,262 describes an ultrasonic sensor that has a thin planar sheet of material forming a Lamb wave propagation medium. Lamb waves, also known as plate-mode waves, can propagate only through a material of finite thickness. In contrast to surface acoustic waves (SAWs), which require a propagation medium having a thickness on the order of hundreds of times the wavelength of the propagating SAW, Lamb waves require a propagation medium which is at most only several wavelengths thick, and typically only a fraction of the wavelength of the propagating Lamb wave. The thickness of the sheet is no greater than about twenty microns. A Lamb wave generator generates Lamb waves in the planar sheet, and an output device produces an electrical signal that represents the propagation characteristics of the Lamb waves propagating along the sheet. A measuring device measures selected characteristics of the output electrical signal. The planar sheet has some physical characteristics that depend upon the value of a measurand acting on the sheet, and those physical characteristics consequently determine the propagation characteristics of the Lamb waves that propagate along the sheet. Since the electrical signal from the output device represents the propagation characteristics, the electrical signal also represents the value of the measurand acting on the sheet.

The Lamb wave device described in U.S. Patent No. 5,129,262 can be employed, for example, in biological sensing. The planar sheet described above can be pre-coated with antibody molecules, so that the frequency of the device changes upon immersion in or contact with a liquid that contains the corresponding antigen. Antigen-antibody attachment at the surface of the propagation medium acts to alter the wave velocity of the Lamb waves in the sheet. The change in wave velocity causes the oscillation frequency to change in a delay line oscillator form of the device. Also, the sheet may be made of a porous and permeable material, allowing the coating of antibody molecules over a greater surface area of the sheet and also allowing the antigen-containing liquid to be flowed through the membrane, in order to speed up the antigen-antibody attachment. Other biological interactions may also be sensed, and additional applications include immunoassay, clinical laboratory testing, in vivo biomedical monitoring, and biomedical research.

The test solutions used in the described embodiment, for example a blocking solution 106, a sample 108, and a buffer 110, are sourced from reservoir containers 112. The channel path from each of the reservoirs 112 is gated with a valve 114 to control the flow of a particular test solution to a combination point 116 leading to an entry port 118 of the FPW device 104. The test solution flows through the FPW device 104 and exits via an exit port 120, which leads to a pump 122. The pump 122 draws the test solution through the network of channels 102 and through the FPW device 104, and directs the test solution to a waste receptacle 124.

FIG. 1B shows another embodiment of the analyte detection system 100. This embodiment packages the FPW device 104 and its associated fluid chamber 160 as a cartridge 103, *i.e.*, a consumable component that can be removed and replaced. Some embodiments may include a fluid control device 101 such as a plug, obstruction or baffle that alters the flow through the device 104. In one embodiment, the fluid control device 101 operates to cause the fluid flow through the device 104 to pass closer to the sensor surface 143 than if the fluid control device 101 was not present. Further, the source of input test solutions is shown as an input fluid chamber 105 that has an outlet 107 for directing the test solutions into the inlet 109 of the cartridge 103. In some embodiments, the magnetic particles are initially located in the input fluid chamber 105 and the fluid containing analyte is mixed with the magnetic particles in the input fluid chamber 105 and then directed into the cartridge 103 in which the FPW device 104 is located. The magnetic particles may be combined within the input fluid chamber 105 with the fluid containing analyte by a device (*e.g.*, by the action of a pump or a magnetic agitator). FIG. 1B further shows an output fluid chamber 111 with an inlet 113 that receives fluid from the outlet 115 of the cartridge 103. This output fluid chamber 111 may include one or more of the fluid control devices described herein, and it may include one or more mechanisms for storing and/or treating waste fluid.

In at least one embodiment, the junction where the outlet 107 of the input fluid chamber 105 meets the inlet 109 of the cartridge 103 is constructed and arranged to allow repeatable connection and disconnection. Similarly, the junction where the outlet 115 of the cartridge 103 meets the inlet 113 of the output fluid chamber 111 is constructed and arranged to allow repeatable connection and disconnection. In some embodiments, these junctions are constructed and arranged to require tools for connection and disconnection, such as threaded couplings that require a wrench or other such tool to affect the coupling and decoupling. In other embodiments, these junctions are constructed and arranged to allow quick and easy manual connection and disconnection, without any extra tools or accessories. Such couplings, both requiring and not requiring tools, are known in the art. In some embodiments, there are multiple input fluid chambers and output fluid chambers. In some embodiments, one or more input and/or output fluid chambers are part of the cartridge 103. Further, in some embodiments, one or more sources of magnetic flux are part of the cartridge.

The FPW device 104 is shown in more detail in FIG. 2 and FIG. 2A. In an FPW device 104, strain energy is carried in bending and tension in the device. In some embodiments, it is desirable for the thickness-to-wavelength ratio of the FPW device 104 to be less than one, and in some cases much less than one. In general, the wavelength "λ" of the FPW device 104 is approximately equal to the pitch of the interdigitated electrodes as described herein. In one embodiment, the thickness-to-wavelength ratio of the FPW device 104 is 2 µm/38 µm. In other embodiments, the FPW device 104 is designed to isolate a particular mode (*e.g.*, any mode from the zero^{th} order mode to higher order modes) or bandwidth of modes associated with the device. For example, an FPW device 104 having a thickness/wavelength of 2 µm/38 µm as described above would isolate the 80^{th} mode of the FPW device 104. The FPW device 104 can be designed to achieve this effect by selecting a particular pattern for the interdigitated electrodes deposited on the device. In one embodiment, the FPW device 104 is rectangular in shape. The FPW device 104 can, alternatively, be circular or elliptical, or some other planar shape.

In general, the FPW device 104 is constructed from a silicon wafer 130, using micro-fabrication techniques known in the art. In the described embodiment, a cavity 132 is etched into the wafer 130 to produce a thin, suspended membrane 134 that is approximately 1.6 mm long, 0.3 mm wide and 2 µm thick. The overall wafer 130 thickness is approximately 500 µm, so the depth of the cavity 132 is just slightly less than the wafer 130 thickness. A 0.5 µm layer 136 of aluminum nitride (AIN) is deposited on the outer surface (i.e., the surface opposite the cavity 132) of the membrane 134, as shown in the expanded view insert of FIG. 2A. Two sets of inter-digitated metal electrodes 138 are deposited upon the AIN layer. A thin layer 140 of gold (approximately 500 angstroms) is deposited on the inner surface (i.e., the surface facing the cavity 132) of the membrane 134 to facilitate immobilization of capture agents (described in more detail below). In some embodiments, the first of the two sets of the interdigitated electrodes is an actuating or launching portion that is located on the left half side (as viewed in FIG. 2) of the membrane 134. The second of the two sets of interdigitated electrodes is a sensing or receiving portion that is located on the right half side (as viewed in FIG. 2) of the membrane 134. In some embodiments, the two sets of the interdigitated electrodes are located side-by-side along the length (viewed from left to right in FIG. 2) of the membrane 134, one of the two sets is the actuating or launching portion and the second of the two sets is the sensing or receiving portion.

In operation, instrument/control electronics 126 (referring to FIG. 1A) apply a time-varying electrical signal to at least one set of electrodes 138 to generate vibrations in the suspended membrane 134. The instrument/control electronics 126 also monitor the vibrational characteristics of the membrane 134 by receiving a sensor signal from at least a second set of electrodes 13 8. When liquid is in contact with the cavity side 132 of the membrane 134, the maximal response of the plate structure is around 15-25 MHz. The instrument/control electronics 126 compare a reference signal to the sensor signal from the second set of electrodes to determine the changes in the relative magnitude and phase angle of the sensor signal as a function of frequency. The instrument/control electronics 126 interpret these changes to detect the presence of the targeted analyte. In some embodiments, the instrument/control electronics also determines, for example, the concentration of the targeted analyte on the inner surface of the membrane 134.

Capture agents targeting the analyte of interest are immobilized on the thin layer of gold 140 covering the inner surface of the membrane 134, as described above. The surface can be coated with a suitable linking compound. Suitable linking compounds are commercially available. In one embodiment, the linking compound comprises biotin PEG disulfide, as described below. In another embodiment, thiol-terminated alkyl chains are linked to the gold surface forming a self-assembled monolayer (SAM). A fraction of the SAM chains are terminated with reactive groups (e.g., carboxyl) to allow covalent linking of capture agents to the SAM chains using biochemical process steps known in the art. The remainder of the SAM chains are terminated with non-reactive groups, preferably ones that have a hydrophilic character to resist nonspecific binding (e.g., oligomers of ethylene glycol). Other surface chemistries are described in the literature and can be used to produce a capture surface.

The FPW device 104 is packaged to allow electrical connections to the electrodes 138 on the outer surface of the membrane 134. Additionally, the FPW device 104 is mechanically supported by a channel block 142, to allow for the inner surface of the membrane 134 to contact the test solutions and an interface is provided for contacting the sensor surface 143 with the liquid sample. The channel block 142 creates a path (fluid chamber 160) for the test solutions to flow from an input port 118, past the inner surface of the membrane 134 and then out of an exit port 120. A seal 144 is formed between the FPW device 104 and the channel block 142 to prevent test solutions from escaping from the channels 102 formed within the combination of the FPW device 104 and the channel block 142. The channel block 142 thus forms a fluid chamber, of which the FPW device 104 comprises one of the interior walls.

The channels 102 through the combination of the FPW device 104 and the channel block 142 are approximately 0.5 mm in diameter. The channel block 142 can be formed from a variety of materials, including plastic, metal or ceramic, among other materials.

The system 100 includes one or more fluid control devices for changing at least one fluid property, such as flow, pressure, or trajectory to name a few, within the system 100. The pump 122 and valves 114 shown in FIG. 1A that direct and control the flows of various test solutions through the device and over the sensor surface 143 (as required to execute a test protocol) are all examples of fluid control devices. In general, a fluid control device changes the at least one fluid property in the vicinity of at least one surface within the fluid chamber 160 of the device 104. Generally, this is done to distribute the magnetic particles along at least a portion of the sensor surface 143. As described above, in some embodiments the fluid control device is a pump (*e.g.*, a peristaltic pump, centrifugal pump, rotary pump, electro-osmotic pump). In some embodiments, the pump is located on the entrance side of the fluid chamber, and in other embodiments the pump is located on the exit side of the fluid chamber. In some embodiments, the device is a flow diverter (e.g., a plug, obstruction wall or baffle) that is disposed relative to the fluid chamber to alter the fluid flow in the vicinity of the at least one interior surface of the fluid chamber.

Referring to FIG. 1A, a single pump 122 is positioned on the waste side of the FPW device 104. Suction that the pump 122 generates draws buffer 110 or analyte in the sample 108 from their respective reservoir containers 112 on the supply side of the FPW device 104. Valves 114 are positioned on the supply side of the device 104 to control which test solution is directed over the sensor surface 143 at any time during the test protocol. The pump 122 controls the flow rate of the test.

A device for regulating temperature (e.g., a thermoelectric cooler) may be associated with the FPW device 104 and channel block 142. This reduces the impact of variable environmental conditions on the FPW device 104 output by maintaining the device 104 at a relatively constant, known temperature. In an alternative embodiment, a temperature sensor is included within the system 100, for example as part of the FPW device 104. The sensor signal from the FPW device 104 is scaled, at a specific instant in time (or during a period of time), based on the output of the temperature sensor, in order to produce a signal that is independent of the effects of temperature variations. This scaling could be done based on a mathematical model, or an analytical model, or some hybrid combination of a mathematical and analytical model.

In some embodiments of the system 100, a filter is included in the path of the test solution to selectively filter particles (*e.g.*, magnetic particles and biological materials) of a particular size to prevent them from entering the fluid chamber. By way of example, a particular testing protocol may include steps for changing the filter during the test. This would allow different types (i.e., sizes) of analytes and magnetic particles to be directed into the fluid chamber, and thereby tested by the system 100, during different portions of the test.

In one embodiment, magnetic particles (*e.g.*, paramagnetic or super-paramagnetic beads or microspheres), which have their surfaces coated with a capture agent, are mixed with a sample containing the analyte. After a prescribed mixing time analyte-particle complexes 146 result as do particles 147 that have bound nonspecific materials and particles 148 that have bound nothing. The particles 146, 147 and 148 are located in the sample reservoir 112.

The system 100 further includes a magnetic field inducing structure 150 for producing magnetic flux in the vicinity of the membrane 134. In FIG. 1A, the source of magnetic flux is a retractable magnet 150 arranged to normally be in close proximity to the membrane 134 of the FPW device 104. When the magnet 150 is in close proximity to the membrane 134, the magnet 150 produces a significant gradient magnetic field in the vicinity of the membrane 134. Under control of the instrument/control electronics 126, the retractable magnet 150 can be retracted away from the membrane 134 by a distance sufficient to substantially reduce magnetic fields in the vicinity of the membrane 134. In one embodiment, when in close proximity to the membrane 134, the magnet 150 is situated approximately 200 µm from the sensor surface 143 of the membrane 134. In another embodiment, when in close proximity to the membrane, the magnet 150 is situated between about 50 µm to about 100 µm from the sensor surface 143 of the membrane 134.

When the magnet 150 is in close proximity to the membrane 134, the magnet 150 provides a source of magnetic flux to draw the magnetic particles from the sample to the sensor surface 143. The analyte-particle complexes 146, as well as particles 147 with nonspecifically bound material and particles 148 with nothing bound, migrate from the liquid sample until they encounter the sensor surface 143. The analyte binds with the capture agent on the sensor surface 143. Thus, the analyte forms a link between the magnetic particle and sensor surface. The particles 147 with non-specifically bound material and particles 148 with nothing bound are held at the sensor surface 143 by the magnetic field. Additionally, weak binding forces can act between the particles 146, 147, and 148 and the sensor surface 143. During the wash step of the protocol (described in more detail below), the magnet 150 is retracted to reduce the magnetic force experienced by the particles that have accumulated at the sensor surface 143. The wash flow rate is increased to remove particles 147 and 148 that are not bound to the surface by analyte. Since the particles 147 with nonspecifically bound material as well as particles 148 with nothing bound are more weakly linked to the sensor surface 143 than the analyte-particle complexes 146, they release from the sensor surface 143 at a lower wash flowrate (and corresponding hydrodynamic force). Hence, removing the magnet 150 (i.e., substantially reducing the magnetic force experienced by the particles 146, 147, and 148 at the sensor surface 143) is used to distinguish between particles with analyte 146 from those without (particles 147 and 148). One technique for engaging and retracting the magnet 150 is to mount it on a carriage (not shown) that is actuated by a cam system (not shown).

The magnet 150 material, geometry and distance from the sensor surface 143 determine the field shape and field gradient, and therefore, the force that the analyte-particle complexes 146 experience. High strength permanent magnets for use as the retractable magnet 150 are available commercially. For example, 1 mm diameter cylindrical NdFeB magnets can be purchased from several vendors (e.g., Dexter Magnetic Technologies). In one embodiment, a 1 mm diameter and 5 mm long NdFeB magnet 150 is positioned within 0.1 mm of the sensor surface 143 when engaged. When retracted the magnet 150 is at least 0.5 mm from the sensor surface 143.
Since the membrane 134 of the FPW device 104 is very thin (2 µm) and made of nonmagnetic materials (e.g., silicon, aluminum nitride or gold), the membrane 134 does not significantly perturb the magnetic field on the sensor surface 143 side of the device 104. As a result, very high magnitude magnetic fields and large field gradients can be achieved, as is necessary for high collection efficiencies.

The sample flow rate through the channels 102 is determined (e.g., specified by an operator) by the residence time necessary for good collection efficiency. The sample flow rate is adjusted so that the average velocity over the sensor surface 143 is between about 1 and about 5 mm/s. With an iron oxide paramagnetic particle with a diameter of approximately 3 µm, collection efficiencies approaching 50% can be achieved.

Other configurations of the source 150 of magnetic flux *(i.e.,* the magnet) may be used. For example, an electromagnet can be used instead of a permanent magnet. The electromagnet includes pole pieces that extend to focus the field flux near the sensor surface 143 of the device 104.

Alternatively, a magnetizable material can be fashioned and positioned adjacent to the sensor surface 143 (within 0.1 mm), and a separate magnet combined with an open face of the magnetizable material to induce a magnetic field in the magnetizable material. The magnetic field induced in the material serves to locate desirable field gradients near the sensor surfaces 143. In this way, large, low cost magnets can be used, and a single magnet can be used to address multiple sensors, depending on the fashioning of the material. Examples of useful materials for this purpose are pure iron, high mu metals such as alloy 49 (high nickel content iron), sna silicon steels (1-2% silicon typical). An advantage of using such a magnetizable material with an associated magnet is that the sensor assembly can be simplified, allowing lower cost manufacturing. A low precision actuator can be used for engaging and retracting the magnet since the magnet need only contact the ferromagnetic core or be fully withdrawn. In the described embodiment where the magnet 150 is positioned in close proximity to the sensor surface 143, a higher level of precision is required to achieve good assay repeatability. Although there is some loss of field strength with this approach, it is still possible to design the overall system to achieve good capture efficiencies (e.g., >10%).

The shape of the tip of the field inducing structure (e.g., magnet or ferromagnetic material) may be tailored to enhance and/or concentrate the field gradient at the surface. Since the size of the FPW device 104 (e.g., 0.3 mm x 1.6 mm) is typically smaller than conventionally formed magnets or machined inductors, the portion of the field inducing structure adjacent to the membrane 134 can be tapered to concentrate the magnetic field in one or more locations on the sensor surface 143. Tapering the tip acts to increase both the local field magnitude and the local field gradients. For example, a wedge-shaped tip is well suited to the current FPW device geometry.

One embodiment of the system 100 includes an optional second source 150a of magnetic flux that opposes or partially opposes the first source 150 of magnetic flux. This second source 150a of magnetic flux can be used to dislodge some of the magnetic particles that have adhered to the sensor surface 143. It may, for example, dislodge magnetic particles 148 that do not have any bound analyte; they would not be as strongly attached to the sensor surface 143 as the particles 146 that do have bound analyte. In some embodiments, the first source 150 of magnetic flux is turned off or moved away from the sensor surface 143 and then, the second source 150a of magnetic flux is positioned relative to the at least one surface of the fluid chamber to selectively remove magnetic particles. This may be done, for example, to remove magnetic particles 148 that do not have any bound analyte and therefore they are not as strongly bound to the sensor surface 143. This would achieve a similar effect as increasing the flow of fluid to remove magnetic particles 148 that do not have any bound analyte.

Controlling the distribution of the analyte-particle complexes 146 on the surface 143 of the device 104 can improve the device performance, since the device 104 has a suspended membrane 134 and not all parts of the membrane 134 contribute equally to the moving mass of the detectable resonance. For example, the system 100 can be constructed and arranged to distribute the analyte-particle complexes 146 within one third of the FPW device 104 width along the middle two-thirds of the centerline of the long axis of the membrane 134. Taking into account flow field effects, the shape of the tip of the field-inducing structure (e.g., magnet 150) can be such that the field magnitude and field gradient increase in the direction of the flow over the sensor membrane 134. That is, analyte-particle complexes 146 in the downstream regions, where the boundary layer is partially depleted of analyte, experience a higher field and field gradient than do analyte-particle complexes 146 in the upstream regions.

In general, the system 100 can be constructed and arranged to concentrate magnetic particles in one or more particular regions of the sensor surface 143. The response of the device 104 may not be uniform over the sensor surface 143 due to characteristics of the fabrication materials or the specifics of the sensor design. Thus, high sensitivity regions of the device 104 may be non-uniform and asymmetrical with respect to the long and short axis centerlines of the device 104. Thus, the tip of the field inducing structure may be shaped to concentrate magnetic particles in the region or regions of highest sensitivity.

Varying the flow rate through the device 104 can also be used to achieve a more uniform coverage of analyte-particle complexes 146 for a given magnetic field distribution. For a given field, magnetic particles interact with the sensor surface 143 as determined by the bulk fluid flow rate, much like a ballistic object might fall in the presence of the gravity body force. In this case, however, the magnetic induced force dominates. By varying the flow rate, the analyte-particle complexes 146 can be caused to interact with the sensor surface 143 at substantially different locations along the stream-wise flow direction. Furthermore, as the magnetic particles pile up (a non-desirable occurrence if they are to be exposed to the sensor surface 143) the flow can be reversed and subsequently pulsed forward in order to pull the pile over and thus communicate more particles with the sensor surface 143. In one embodiment of the system 100, selective location of the magnetic particles along the sensor surface 143 is achieved by selectively altering, over the course of the detection protocol, either one or both of the magnetic flux source and the property or properties of the fluid flow along the sensor surface 143.

One embodiment of the system 100 includes a device (*e.g.*, optical, magnetic) for characterizing at least one property of the magnetic particles that are attached or attracted to the sensor surface 143. This device could be an integral part of the FPW device 104, or it could be a part of the magnet 150, or it could be a discrete component apart from other components of the system 100. Such a device may be used to detect the presence of the particles, and also to determine parameters related to the particle, for example, the size, quantity, concentration, or density of the particles that are attracted to the sensor surface 143.

One embodiment of the system 100 includes an identification device for allowing an operator or computer to identify the system 100 or a particular component of the system for tracking usage of the system or component. The identification device may include a symbol or image such as a bar code, an identification number, or other identifying mark. The identification device may include an actual component, passive or active, such as an RFID tag, an integrated circuit or other such component known in the art for providing identifying information. Many such devices are known in the art, although any contemplated identification device may be used.

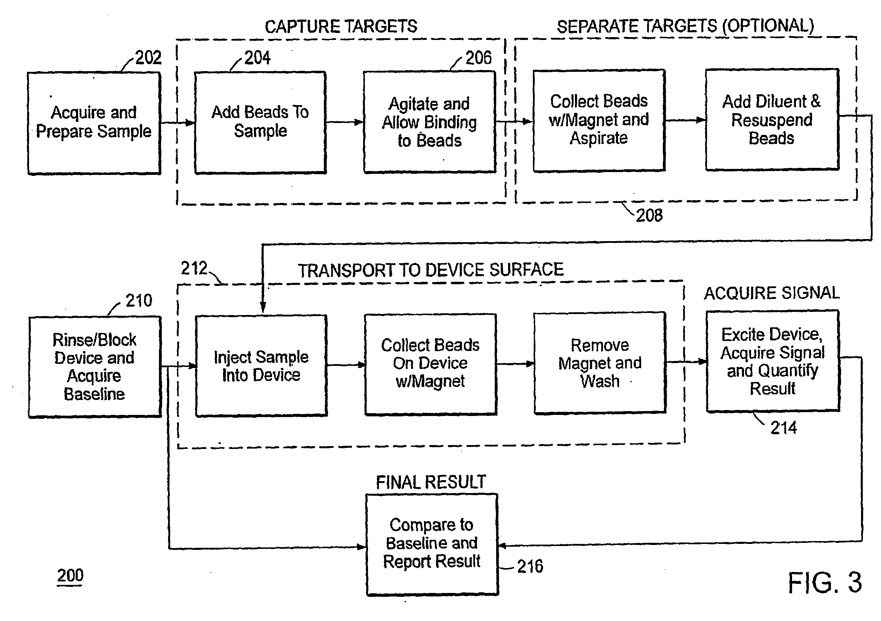

A general detection protocol 200 for using an FPW device 104 in combination with analyte-particle complexes 146 for detection of biological analytes is shown in FIG. 3.

The first step 202 of the detection protocol 200 is acquiring and preparing 202 the analyte sample. Various preparation processes may need to be performed prior to testing, depending upon the particular type of analyte being tested. For example, to detect microbes in food (*e.g.*, E. coli in ground beef), a sample would be first mixed with enrichment broth, stomached, incubated and filtered. For detecting proteins in blood, the sample would first be filtered or centrifuged and the serum separated. Specific examples of test protocols that include sample preparation steps are described herein.

The next step 204 of the detection process is mixing affinity-coated paramagnetic particles (i.e., beads) with the prepared analyte sample. Paramagnetic or super-paramagnetic particles are available commercially from a number of vendors (e.g., Dynal Biotech, Oslo, Norway). Typical diameters range from 50 nm to 10 µm, and such particles can be procured already coated with affinity agents (e.g., antibodies, proteins, and nucleic acid probes) targeting a variety of analytes (e.g., cells, proteins and nucleic acids). Alternatively, the particles can be purchased with various reactive chemical groups (e.g., epoxy, carboxyl, and amine) in order to attach a capture agent of choice. Standard biochemical protocols are available for this purpose.

The sample with paramagnetic particles added is agitated 206 for an amount of time determined by the particular analyte and capture agent. During this process, the particles bind with analyte so that the analyte is captured on the particles. In some cases, the sample can be tested directly at this point. But, in other cases it is advantageous to perform separation steps 208 to isolate the analyte bound to the particles from the rest of the original sample. These separation steps 208 reduce interference from other biological material in the assay. Manual or automated equipment for performing such separation steps is available commercially (e.g., Dexter Magnetic Technologies, Dynal Biotech). The basic process uses a magnet to localize the paramagnetic particles on a surface so that the majority of the sample liquid can be aspirated. The magnet (e.g., magnet 150 of FIG. 1A) is then removed, and clean buffer solution is added to re-suspend the particles.

In one embodiment, a baseline step 210 is executed prior to testing the processed analyte sample with the FPW device 104. During the baseline step 210, a reference solution 106 is flowed through the system to rinse/block the sensor 104, and the instrument/control electronics 126 excite the device 104 and records the resulting initial baseline signal from the device 104.

A sample transport step 212 follows the baseline step 210. The sample 108 containing the analyte-particle complexes 146 is flowed over the sensor surface 143 with the magnet 150 engaged. Analyte-particle complexes 146 are collected on the sensor surface 143. After a prescribed volume of sample 108 has flowed through the device 104, the magnet 150 is retracted to release the particles 147 and 148 from the sensor surface 143 that do not have bound analyte, and the flow is switched to a wash solution (e.g., buffer solution 110). The flow rate of the wash solution is increased to help remove loosely bound particles 147 and 148, as well as other material in the sample that may have bound to the sensor surface 143

An acquisition step 214 follows the sample transport step 212. Reference solution 106 is again run through the device 104, and the instrument/control electronics 126 excite the device 104 to acquire and record a final baseline signal from the device 104.

The system 100 determines the amount of analyte accumulated on the sensor surface 143 during the transport step 212 by comparing 216 the initial baseline signal and the final baseline signal, which correspond to the vibrational characteristics of the FPW device 104 in the reference solution before and after, respectively, the acquisition step 214. Analyte-particle complexes 146 bound to the sensor surface 143 change the vibrational characteristics of the FPW device 104, and the amount of change to the vibrational characteristics correspond to the amount of analyte-particle complexes bound to the sensor surface 143.

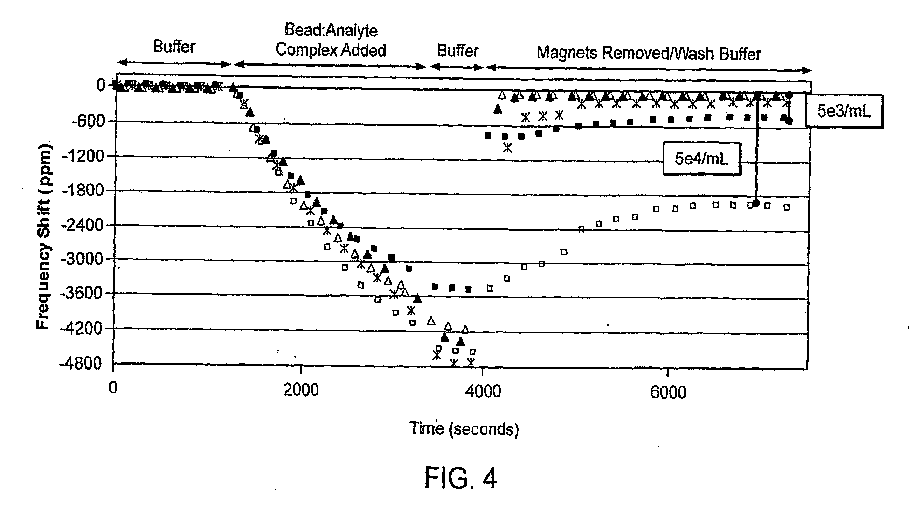

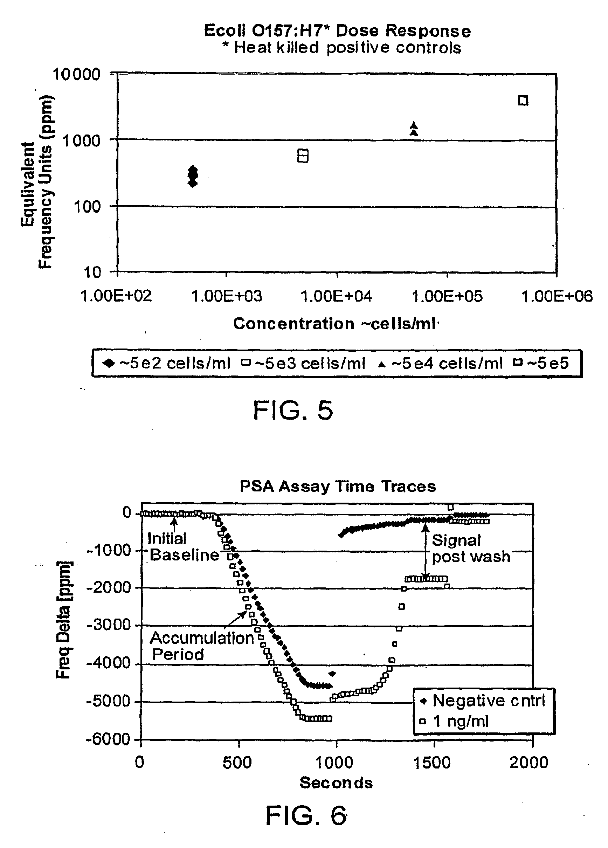

FIG. 4 shows the change in the signal from multiple FPW devices 104 as a function of time for an exemplary detection protocol. The square symbols correspond to a device 104 exposed to an analyte; the triangles and stars correspond to negative controls (in which there is no analyte on the beads). The data shown in FIG. 4 (and FIG. 5 as described below) represent an exemplary detection protocol for which the analyte is E. coli bacteria, or generally a cellular analyte.

In this particular experiment, the frequency of a resonant peak was tracked. FIG. 4 shows that the resonant frequency of the FPW device 104 decreases as the magnetic particles 146, 147 and 148 accumulate on the surface. Once the magnet 150 is removed and some of the magnetic particles are washed away, the frequency of the device 104 increases. Eventually, the system establishes a final baseline that can be compared to the initial baseline taken at the start of the test, or to that of a control.

FIG. 5 is a summary plot showing the final signal change detected as a function of original analyte concentration.

Other detection protocols may be used with the system 100 described above. Individual steps can be eliminated or added depending on the requirements of a specific application or analyte. For example, FIG. 6 shows a time evolution plot for a detection protocol, similar to the one shown in FIG. 4. FIG. 6, however, depicts a detection protocol for a PSA assay, which demonstrates the capability of the system 100 for detecting proteins. Also, the flow direction can be reversed during the protocol. For example, reversing the flow direction may be useful for washing nonspecifically bound material from the device, or for making more efficient use of the available sample.

Another variation in the detection protocol includes alternating between wash steps and binding steps. This can allow better use of the dynamic range of the device. In come cases, a large fraction of the particles do not have bound analyte, especially at low analyte concentrations. By repeatedly binding and washing away particles, it is possible to accumulate more analyte-particle complexes 146 and, hence, improve the sensitivity of the measurement.

Changing or manipulating the magnetic field distribution at the sensor surface 143 during the transport step 212 can enhance the probability that the analyte attached to a particular particle encounters the sensor surface 143. For example, if the spatial distribution of the field is alternated during binding, it is possible to cause the paramagnetic particles at the sensor surface 143 to roll. In some embodiments, by controlling the spatial distribution of the field, an operator or the instrument/control electronics 126 can be used to control the rolling of the paramagnetic particles along the sensor surface 143.

As described above, introducing a second magnetic field (i.e., a second source of magnetic flux) in the system 100 can improve the control of the assay conditions and enhance the specificity of the assay. For example, during the binding or wash steps of the protocol as described above, applying a secondary magnetic field to the sensor surface 143 can act to pull off weakly bound magnetic particles. The strength of the secondary field can be tailored to generate a force on the analyte-particle complexes 146 at the sensor surface 143 that is below the binding force of the specifically bound analyte but above the typical binding force for nonspecifically bound material. This sequence of steps can be repeated multiple times during the assay to further increase the specificity of the test.

The relative binding strength of the various analyte-particle complexes 146 on the sensor surface 143 can be determined by increasing (continuously or discretely) this magnetic pull-off force during the wash step, while monitoring the response of the FPW device 104. Information on the relative binding strength can be used to distinguish between different analytes in the sample 108.

The particular way the sample interfaces with the device 104 can be different in other embodiments. In the above-described embodiments, the system 100 flows the sample through a channel to establish contact between the analyte-particle complexes 146 and the sensor surface 143. In an alternative variation of the system 100, the FPW device 104 is mounted on a probe and at least partially immersed into a test solution containing magnetic particles bound to an analyte. For this embodiment, the immersion is sufficient to place the bound magnetic particles in proximity to the sensor surface 143 so that the particles are attracted toward the sensor surface 143 and subsequently detected, as described herein. To obtain a baseline signal, the device 104 (or cartridge 103) is immersed in a reference test solution. In some embodiments, a portion of the device 104 (e.g., the membrane 134) is mounted to a probe. Further, only part of the sensor surface 143 of the membrane 134 is placed in contact with the solution containing magnetic particles bound to an analyte. In these embodiments, immersion or controlled movement of the probe in the fluid is sufficient to place the bound magnetic particles in proximity to the sensor surface 143 so that the particles are attracted toward the sensor surface 143 and subsequently detected.

Another alternative embodiment of the system 100 involves mounting the device 104 inside of a tube that can be partially immersed into a well holding the sample 108, and then retracted. A pump applies suction to draw sample into the tube and over the sensor surface 143 (or cartridge 103) when the sample is immersed. The sample is then ejected back into the well by reversing the pump or simply by venting the tube. This cycle of drawing and releasing the sample can be repeated to improve the collection efficiency and, therefore, the performance of the assay.

The following examples illustrate, for one embodiment of the system 100 described herein, steps for preparing and utilizing the system 100 for detecting an analyte. Example I Generalized Method for Capture Agent Functionalization of a Surface of a Flexural Plate Wave Device

1. Deposit gold onto the surface (e.g., sensor surface 143) of the flexural plate wave device 104 and clean the gold surface 143 with, for example, oxygen plasma.

2. An ideal surface chemistry for the surface 143 of the gold is one that provides 1) non-specific binding resistance and 2) reactive groups located on the surface for covalent attachment of capture agents. An exemplary surface chemistry for the surface 143 of the gold is a self-assembled monolayer (SAM) of alkane thiols. The SAM can be formed from a mixture of two alkane thiols; one terminated with a reactive group for subsequent covalent attachment of capture agents, and one terminated with a non-reactive group. By way of example, a mixture of EG₃-OH (EG3) and EG₆-OCH₂COOH (EG6) terminated C₁₁-alkane thiols may be used for this purpose. In one embodiment, the flexural plate wave device 104 (particularly the surface 143 of the device 104) is placed in contact with the alkane thiol solution and allowed to incubate at room temperature for, for example, about 16 hours. The surface 143 of the flexural plate wave device 104 is then rinsed with ethanol and blown dry with nitrogen.

3. The next step involves covalent attachment of capture agent or analyte to the surface 143 of the flexural plate wave device 104. A number of methods may be used for covalent attachment of capture agent or analyte. An exemplary method involves covalently linking a biotin linker moiety to the SAM, then binding a biotinylated antibody or analyte to the flexural plate wave device surface 143 via a streptavidin linking layer.
Example II Detecting E. coli 0157:H7 In Ground Beef using, for example, the method of FIG. 3 (FIG. 5 contains data representative of various concentrations of E. coli).

1. Prepare an analyte sample containing E. coli O157:H7 with a concentration greater than about 100 cfu/mL.

a. Concentrate the analyte sample in solution by performing an immunomagnetic separation. A variety of commercial instruments (e.g., PATHATRIX™ (antibody coated paramagnetic particles) by Matrix Microsciences and BEAD RETRIEVER™ (magnetic bead processor) by Dynal Biotech) or manual methods may be used to perform the immunomagnetic separation. An exemplary manual method involves:

b. Resuspend magnetic beads coated with E. coli antibody (e.g., Dynabeads anti-E. coli 0157, available from Dynal Biotech) until the magnetic bead pellet in the bottom of the tube disappears. Place a microcentrifuge tube in the rack (e.g., a Dynal MPC-S) of a magnetic plate. Pipette 1-20µL of magnetic bead stock solution into the tube (the volume of magnetic bead stock selected is based on desired final bead concentration).

c. Add 1 mL of the analyte sample to the tube in the rack of the magnetic plate and close the tube.

d. Invert the tube a few times. Incubate the solution in the tube at room temperature for 10 to 60 minutes with gentle continuous agitation to prevent magnetic beads from settling.

e. Invert the tube several times to concentrate the magnetic beads into a pellet on the side of the tube. Allow about 3 minutes for proper recovery.

f. Open the tube and carefully aspirate and discard the sample supernatant as well as the remaining liquid in the tube's cap.

g. Remove the magnetic plate.

h. Add 1 mL of wash buffer (PBS-TWEEN® (polyoxyethylene sorbitan monolaurate)) to the tube. Close the cap of the tube and invert the rack a few times to resuspend the beads.

j. Repeat steps e-h twice.

k. Mix the contents of the tube briefly using a vortex mixer.

2. Detection of E. coli O157:H7

a. Functionalize (similarly as described by the steps in A.3 and A.4) the surface 143 of a flexural plate wave device 104 with E. coli O157:H7 antibody.

b. Place a first inlet hose into a tube containing standard wash buffer (1xPBS with 0.05% TWEEN® 20 (polyoxyethylene sorbitan monolaurate)) and a second inlet hose into the tube containing the magnetic beads bound with E. coli O157:H7 (prepared in B.2). The first inlet hose and second inlet hose are joined by a t-joint so the two hoses are in fluid communication and fluid from either the first inlet hose or from the second inlet hose is directed to an inlet of the fluid chamber 160. Each of the two hoses has a valve that is capable of permitting or limiting the flow of fluid through respective hoses.

c. Place a first outlet hose in fluid communication with an outlet of the fluid chamber 160. Fluid from the first outlet hose is collected in a waste collection bottle.

d. A baseline output signal is obtained using the flexural plate wave device. The baseline signal is measured with standard wash buffer flowing into the fluid chamber 160 and out of the fluid chamber 160 for about 5 minutes at a standard pump speed (e.g., 200 uL/min).

e. The first source of magnetic flux 150 is engaged, and then fluid from the tube containing the analyte is directed into the inlet of the fluid chamber 160. Fluid is directed into the inlet of the fluid chamber 160 to accumulate magnetic beads bound with E. coli O157:H7 on the at least one surface 143 of the flexural plate wave device 160 until a desired amount is attached to the at least one surface 143. In one embodiment, the desired amount is achieved when, for example, there is a frequency shift in the output of the flexural plate wave device 104 of about 4000 ppm.

f. The flow of fluid from the tube containing the analyte is then discontinued. Fluid from the wash buffer tube is then directed into the fluid chamber 160 to wash away nonspecifically bound material (i.e., materials other than 1) magnetic beads and 2) magnetic beads with bound analyte).

g. The first source of magnetic flux 150 is then disengaged.

h. Initiate automatic wash protocol to remove any magnetic beads or matrix components.

i. The final signal output by the flexural wave plate device 104 is then measured. The baseline signal is compared with the final signal to determine the concentration of E. coli O157;H7 in the analyte sample.

### Example III Detecting Prostate Specific Antigen (PSA) In Human Blood Serum using, for example, the method steps of FIG. 3.

1. Prepare an analyte sample containing human serum obtained by centrifugation from a human blood sample.

2. Concentrate the analyte sample in solution by performing an immunomagnetic separation. A variety of commercial instruments (*e.g.*, PATHATRIX™ (antibody coated paramagnetic particles) by Matrix Microsciences and BEAD RETRIEVER™ (magnetic bead processor) by Dynal Biotech) or manual methods may be used to perform the immunomagnetic separation. An exemplary manual method involves:

a. Resuspend magnetic beads coated with PSA antibody (e.g., Dynabeads anti-PSA, available from Dynal Biotech) until the magnetic bead pellet in the bottom of the tube disappears. Place a microcentrifuge tube in the rack (e.g., a Dynal MPC-S) of a magnetic plate. Pipette 1-20µL of magnetic bead stock solution into the tube (the volume of magnetic bead stock selected is based on desired final bead concentration).

b. Add 1 mL of the analyte sample to the tube in the rack of the magnetic plate and close the tube.

c. Invert the rack a few times. Incubate the solution in the tube at room temperature for 10 to 60 minutes with gentle continuous agitation to prevent magnetic beads from settling.

d. Invert the rack several times to concentrate the magnetic beads into a pellet on the side of the tube. Allow about 3 minutes for proper recovery.

e. Open the tube and carefully aspirate and discard the sample supernatant as well as the remaining liquid in the tube's cap.

f. Remove the magnetic plate.

g. Add 1 mL of wash buffer (PBS-TWEEN® (polyoxyethylene sorbitan monolaurate)) to the tube. Close the cap of the tube and invert the rack a few times to resuspend the beads.

h. Repeat steps d-g twice.

i. Mix the contents of the tube briefly using a vortex mixer.

3. Detection of PSA

a. Functionalize (similarly as described by the steps in A.3 and A.4) the surface 143 of a flexural plate wave device 104 with PSA antibody.

b. Place a first inlet hose into a tube containing standard wash buffer (1xPBS with 0.05% TWEEN® 20 (polyoxyethylene sorbitan monolaurate)) and a second inlet hose into the tube containing the magnetic beads bound with PSA (prepared in B.2). The first inlet hose and second inlet hose are joined by a t-joint so the two hoses are in fluid communication and fluid from either the first inlet hose or from the second inlet hose is directed to an inlet of the fluid chamber 160. Each of the two hoses has a valve that is capable of permitting or limiting the flow of fluid through respective hoses.

c. Place a first outlet hose in fluid communication with an outlet of the fluid chamber 160. Fluid from the first outlet hose is collected in a waste collection bottle.

d. A baseline output signal is obtained using the flexural plate wave device 104.
The baseline signal is measured with standard wash buffer flowing into the fluid chamber 160 and out of the fluid chamber 160 for about 5 minutes at a standard pump speed (e.g., 200 uL/min).

e. The first source of magnetic flux 150 is engaged, and then fluid from the tube containing the analyte is directed into the inlet of the fluid chamber 160. Fluid is directed into the inlet of the fluid chamber 160 to accumulate magnetic beads bound with PSA on the at least one surface 143 of the flexural plate wave device 104 until a desired amount is attached to the at least one surface 143. In one embodiment, the desired amount is achieved when, for example, there is a frequency shift in the output of the flexural plate wave device 104 of about 4000 ppm.

f. The flow of fluid from the tube containing the analyte is then discontinued. Fluid from the wash buffer tube is then directed into the fluid chamber 160 to wash away nonspecifically bound material (i.e., materials other than 1) magnetic beads and 2) magnetic beads with bound analyte).

g. The first source of magnetic flux 150 is then disengaged.

h. Initiate automatic wash protocol to remove any magnetic beads or matrix components.

i. The final signal output by the flexural wave plate device 104 is then measured.
The baseline signal is compared with the final signal to determine the concentration of PSA in the analyte sample.

### Example IV: PSA in Calibrator I

By way of illustration, an experiment was conducted in which data was acquired using the system 100 of FIG. 1A, according to principles of the present invention. Dynal tosyl-activated super paramagnetic beads, functionalized with anti-Prostate Specific Antigen (PSA) capture antibody (PN 90205, Scripps Laboratories Inc. with offices in San Diego, CA) were exposed to samples. The samples comprised 1 x PBS (Phosphate Buffered Saline) and 1% Bovine Serum Albumin (BSA), spiked with approximately 0pg/mL 10pg/mL, 100pg/mL and 500pg/mL of free PSA [Fitzgerald Industries International, Inc. with offices in Concord, MA]. A bead concentration on the order of approximately 2x10⁴ beads/mL with respect to sample was used in the experiment. Spiked samples where incubated with beads with gentle continuous agitation for 1 hour.

Eight of the Flexural Plate Wave (FPW) devices 104 of FIG. 2 provided on a single chip in a cartridge (not shown) were functionalized with complimentary anti-PSA antibodies (PN 90197, Scripps Laboratories Inc. with offices in San Diego, CA) after being first primed with 1 x PBS containing 0.05% TWEEN® 20 (polyethylene glycol sorbitan monolaurate) [Sigma-Aldrich Co. with offices in St. Louis, MO].

Data was acquired and analyzed as described. for example, in the U.S. Patent Application entitled "Methods and Apparatus for Assay Measurements" by Masters et al. filed on May 2, 2006 (Attorney Docket Number BIO-008). A baseline measurement (similarly as described previously herein) of eight individual frequencies, each corresponding to tracked sensor phases, each with respect to reference signals, was made at about 17800 seconds. Tracking phases are initially selected for each device to be within a resonance band of the device, near a frequency where the magnitude of response is near a peak value and where the phase response has significant linear range with respect to frequency change. When tracking the sensor phases, at each time point, individual device tracking frequencies are found by 1) sweeping each device over a range of frequencies and recording the phase of response at each excitation frequency with respect to a reference signal, 2) fitting a function relating excitation frequencies to measured phase for each device, and 3) using that function to compute the tracking frequency corresponding to the previously determined tracking phase.

In this embodiment, the devices are operated near 20 MHz and the sweep range is approximately 20kHz. Over this range, the phase characteristic is substantially linear allowing the fit function to be linear. The reference signal for each device comprises the output of a network of passive electrical components, resistors and capacitors, simultaneously driven by the excitation. The reference network is selected to match the attenuation and provide a preferred phase shift for the devices near resonance. Baseline frequencies are referenced to, and normalized by, the tracked frequency and are shown as parts per million (ppm) at a selected point in time.

The sensing surface 143 of each device 104 was functionalized with capture agent.
Gold coated chips were cleaned using an oxygen plasma source in which typical processing conditions were about 50 W for about 2 minutes. The chips were subsequently immersed in pure ethanol for 30 minutes. Next, the chips were transferred to a 0.5 mM solution of biotin PEG disulfide solution (Cat No. 41151-0895, Polypure AS with offices in Oslo, Norway) in ethanol and allowed to incubate overnight. The chips were transferred back into a pure ethanol solution for 30 minutes. The chips received a brief, final ethanol rinse and were blown dry using a nitrogen stream. Variations on preparation conditions can be made with similar results achieved.

The resultant biotinylated surface of the devices 104 was coated with Neutravidin (PN 31000, Pierce Biotechnology, Inc. with offices in Rockford, IL) by flowing a 10µg/ml solution of neutravidin over the biotinylated surface for 1 hour. Antibody was biotinylated according to the manufacturer's instructions (PN F-6347, Invitrogen Corporation with offices in Carlsbad, CA) and then coupled to the neutravidinated surface, by flowing 5 µg/ml solution of the biotinylated antibody (diluted into 1x PBS 0.1% BSA buffer), over the neutravidin coated surface for 1 hour.

PSA sample was introduced and simultaneously a magnetic field was generated near the sensor surfaces 143 from about 17900 to about 18200 seconds. Samples of approximately 0pg/mL, 10pg/mL, 100pg/mL and 500pg/mL of free PSA were each provided to two different devices 104 (total of eight devices). The samples were flowed over the sensors at approximately 100 µL/min for a total sample run/volume of 500 µL flowing over the sensors. In this manner, Dynal tosyl-activated super paramagnetic beads coated with free PSA were bound to a substantial surface (surface 143) of the devices 104. Each bead was bound to the surface 143 of the devices 104 by a plurality of bonds. Each plurality of bonds giving rise to the discriminatory force with which each binds to the surface of the device. The characteristic of association and dissociation of an ensemble of beads for each sample determines the concentration of analyte in that sample.

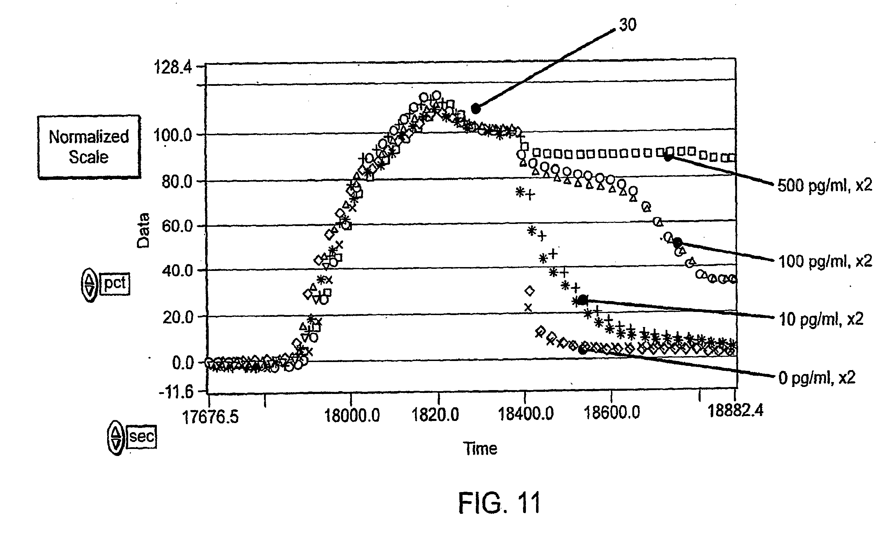

At approximately 18200 seconds the sample was replaced with priming buffer (1 x PBS, 0.05% TWEEN® 20 (polyethylene glycol sorbitan monolaurate)). As shown in FIG. 11, a change (see location 30) in each of the signals was observed from 18200 seconds to 18400 seconds and represents the change in bulk fluid properties associated with switching from the sample fluids back to the buffer fluid. At approximately 18400 seconds the magnetic field was disengaged. The flow speed of the buffer fluid (1 x PBS containing 0.05% TWEEN® 20 (polyethylene glycol sorbitan monolaurate)) was approximately 50uL/minute between about 18200 seconds and 18400 seconds (corresponding to a flow speed of approximately 1.5 mm/second at the sensor surface 143).

The flow speed was increased over the next 450 seconds (from about 18400 seconds to about 18850) from approximately 50 µL/min to approximately 300 µL/min in linear increments (the flow speed was increased by about 17 µL/min every 30 seconds for 450 seconds then the flow speed was reduced to approximately 50µL/min). In this manner, a controlled external influence (i.e., the flow speed in this embodiment) was applied to the beads by increasing the flow speed between about 18400 and about 18850 seconds). The portion of curves between about 18400 and about 18850 seconds represents a signal that is indicative of the change in the amount of beads (and other non-specific material) bound to the sensor surfaces 143 over that time period.

FIG. 11 is a graphical illustration of a plot of the data acquired versus time. The Y-Axis of the plot is the change in relative magnitude of the signal output by the device 104 (in parts per million) at a tracked sensor phase near the resonance of the device 104. The X-Axis of the plot is time in units of seconds. The plot is the change in tracked frequency referenced to and normalized by a tracked frequency at a selected point in time.

The data for each curve shown in parts per million of tracked frequency, was further normalized by the value of that curve at about 18350 seconds and referenced to the baseline frequency that was measured before introduction of the sample fluid. Each data curve was thereby scaled to a value of 100% at about 18350 seconds compared to when sample is introduced. The data associated with each of the normalized curves was then integrated with respect to time over the 450 seconds (from about 18400 seconds to about 18850 seconds) after the magnetic field was removed at about 18400 seconds.

The integration was performed by accumulating respective device signal levels, each interval level multiplied by the respective interval time periods, and dividing the final sum by the period over which the sum was performed. This provides the time normalized amount of material elements (beads) bound to the surfaces 143 of the devices, and these values are shown here to be a measure of concentration of analyte associated with each sample. In this manner, the concentration of analyte is determined based on the change in the amount of material elements bound to the surfaces 143 of each device 104 during the period of time between about 18400 seconds to about 18850 seconds. As shown in FIG. 11, approximately 10 pg/mL of free PSA was detected within less than about 9 minutes of introducing the sample into the system.

In some embodiments, data points are omitted that are, for example, outside the normal variation observed in the curves. For example, spurious data points that vary by more than an order of magnitude in value relative to adjacent data points may be omitted from subsequent analysis. The data points may be removed from the data by, for example an operator or by a computer program.

### Example V: Competitive Assay for Estradiol in Serum

A sheep monoclonal antibody specific for free estradiol was coupled to Dynal M280-tosyl activated beads using standard methods.

A sensor surface was constructed by coupling disulphide-PEG-biotin to the gold sensor surface, then coupling Neutravidin (Pierce PN 31000) and then biotinylated antibody as described above.

BSA coupled estradiol, E2-6-CMO-BSA (Sigma PN E5630) was then coupled to the antibody surface by flowing 10 ug/ml BSA coupled estradiol diluted in 1 x PBS buffer over the antibody surface for 1 hour. Since the estradiol is coupled to multiple amine sites on the BSA (30 mols of estradiol per mol of BSA) the aforementioned approach provides for a moderately dense small molecule surface presented to solution.

Sample solutions comprising 70% charcoal striped human serum (Texas Biologicals), 30% 1 x PBS 0.05% TWEEN® 20 (polyethylene glycol sorbitan monolaurate), were made with free estradiol (Sigma) spiked in at 0 pg/ml, 10 pg/ml, 30 pg/ml, 200 pg/ml. Beads, in concentration ∼2x10⁴/ml were incubated with sample for 2 hrs. The beads were then flowed over the FPW using typical protocol and results obtained in approx 0.5 hrs (similarly as described herein).

In some samples danazol was also added as an decomplexing antagonist, to remove any interfering molecules such as Sex Hormone Binding Protein from the estradiol. Approximately 10-100 ng/ml levels were added.

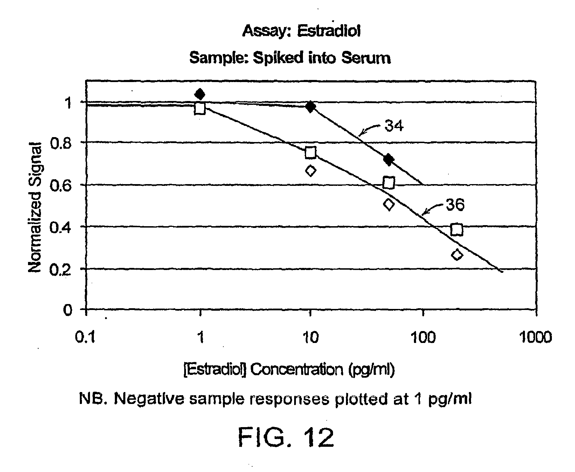

Sample was flowed over the FPW at approximately 70 uL/min with magnetic field engaged and beads were accumulated on the sensor surfaces. A wash protocol beginning at 50 ul/min, ending at 500 ul/min, with 50 ul/min intervals every 30 seconds was used to wash the bound beads off the sensor surfaces. FPW signals were normalized by exposure levels giving normalized signals, and these signals were integrated over the wash period. As shown in FIG. 12, less than 10 pg/ml can be detected in the presence of the antagonist additive 36, and less than 50 pg/ml can be detected in the absence of the antagonist additive 34.

### Example VI: competitive assay for FK-506

1. FK-506 was added to buffer (0.05% PBS TWEEN® 20 (polyethylene glycol sorbitan monolaurate) containing 1% BSA) to produce analyte samples with FK-506 concentrations of 0, 0.5, 2.0, and 20 ng/ml.

2. Eight µl of 1 x 10⁷/ml anti-FK-506 IgM coated beads prepared as described above were added to each sample and incubated for 30-60 min. at room temperature. The beads are prepared by coupling anti-FK-506 IgM (Fitzgerald Industries Intl) to paramagnetic beads according to the manufacturer's instructions (Invitrogen Corporation). The analyte samples were concentrated in solution as described above.

3. Five hundred µl of a solution comprising carrier (HRP) labeled with FK-506 (Diasorin Inc.) were added to the sample and incubated for 60 min. at room temperature.

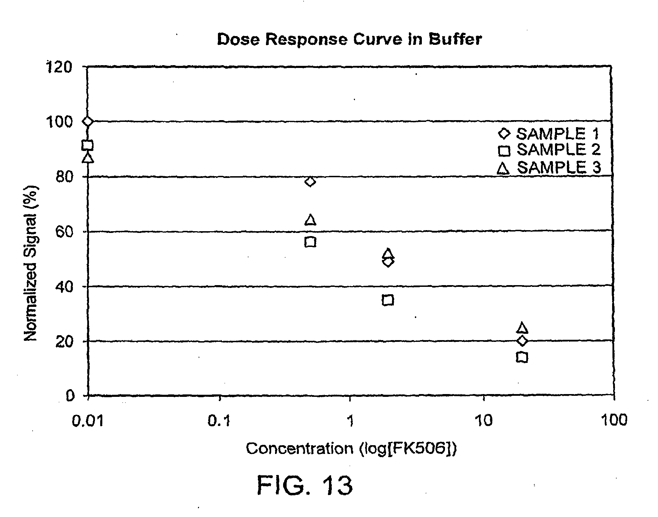

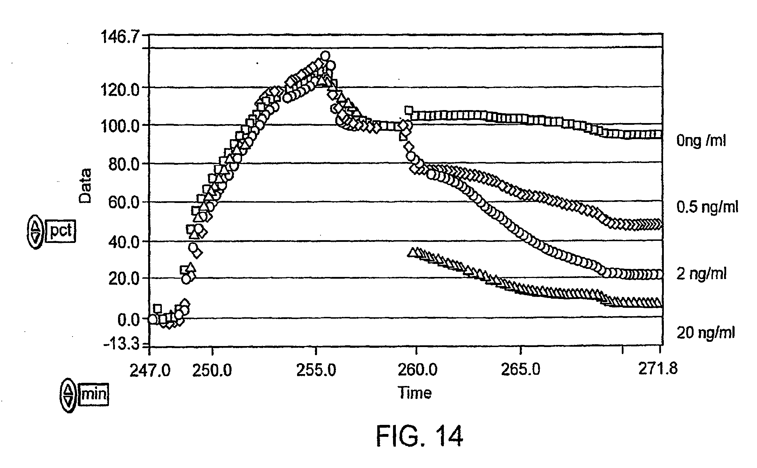

4. The samples were analyzed using an FPW device where the sensor was functionalized with capture agent (anti-FK-506 IgM). Referring to FIG. 10, the sensor 12 was functionalized with neutravidin 30 as described above, and anti-FK-506 IgM was biotinylated 32 using standard biotinylation methods. As shown in panel B of FIG.10, analyte 10 (in this instance FK-506) was mixed with competitor molecule 24 and particles 16 labeled with capture agent 14 (in this instance anti-FK-506 antibody). For this example, the competitor molecule 24 comprised the carrier HRP labeled with the analyte FK-506). As shown in panels C and D, a lower level of FK-506 in the sample is expected to allow the particles to bind to the competitor molecule and thereby to bind to the sensor surface. A higher level of FK-506 is expected to result in fewer particles binding to the sensing surface because more of the particles will be bound to FK-506 from the sample. As shown in FIGS. 13 and 14, FK-506 concentrations ranging from 0.5 ng/ml to 20 ng/ml were detected in as little as 15 min from introducing the sample into the device (similarly as described herein).

### Example VII: competitive assay for FK-506 in blood

1. Four samples each of one 100 µl of blood was spiked with 0, 3,10, or 30 ng/ml FK-506. The drug was extracted from the blood matrix by performing a protein digestion protocol according to the manufacturer's instructions (Diasorin Inc.).

2. Six hundred microliters of protein digestion reagent was added to each sample.

3. The sample was mixed by vortexing, and incubated for 15 min. at room temperature (about 23 ° C), producing a semi-transparent mixture.

4. The digestion reaction was stopped by incubating the samples at 75 ° C for 35 min, producing a dark brown mixture.

5. The sample was mixed by vortexing and centrifuged at 1800g for 10 min, producing 500-600 µl of supernatant.

6. Five hundred microliters of supernatant was transferred into a new tube, the four samples the same concentration of FK-506 were combined in one tube, producing 2 ml of supernatant for each concentration of FK-506.

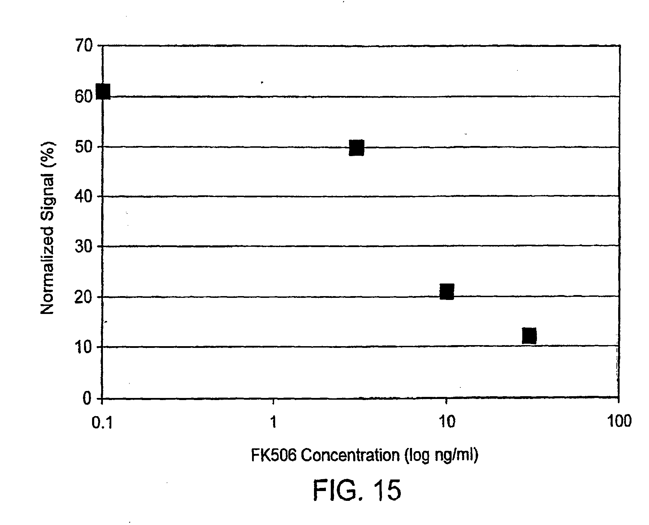

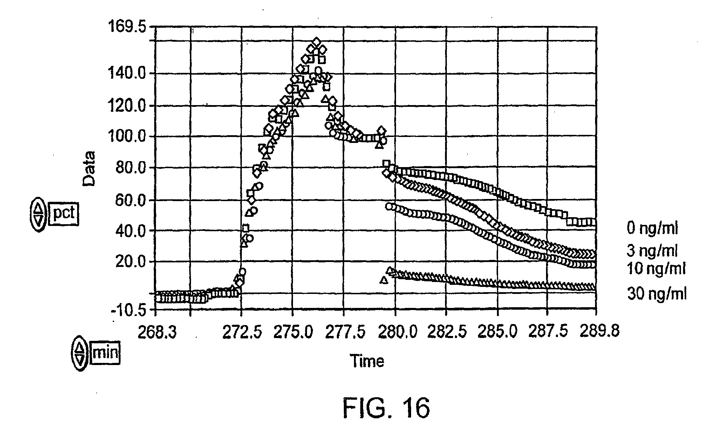

7. The samples were analyzed as described above. As shown in FIGS. 15 and 16, FK-506 concentrations ranging from 0.5 ng/ml to 20 ng/ml were detected in as little as 8 min from introducing the sample into the device.

### EXAMPLE VII cTroponin in buffer

Three monoclonal capture antibodies (Fitzgerald 10-T79 - clone numbers M8010521, M0110609 and M0110510) were coupled to Dynal M280 Tosyl activated beads in the ratio 2:2:1 respectively. Monoclonal Ab, 10-T79B, clone number M8010509 was biotinylated with the Invitrogen labeling kit F-6347 and coupled to gold coated FPW sensors using the Polypure biotin PEG disulphide linker (as described above), subsequently modified with Neutravidin (Pierce PN 31000).

Troponin I (cardiac) antigen was diluted into 1 x PBS 1% BSA samples in the concentrations 0 ng/ml, 0.2 ng/ml, 1 ng/ml and 5 ng/ml. Beads (also referred to herein as particles) were added in the concentration of ∼2 x 10⁴/ml and samples incubated for 7-10 mins before been drawn to the sensor (3-5 mins), followed by a 5 min wash. The results are shown in FIG. 18. Background levels pertaining to samples containing 0 ng/ml of analyte are plotted at 0.01 ng/ml for reference.

### EXAMPLE VIII cTroponin in charcoal stripped serum

Samples were prepared with 70% charcoal stripped serum, 30% phosphate buffered saline 0.05% TWEEN® 20 (polyethylene glycol sorbitan monolaurate). Cardiac troponin I (Biospacific) was added to final concentrations 0 ng/ml, 0.4 ng/ml, 1 ng/ml and 5 ng/ml. Beads were functionalized with anti-cTroponin antibody (Biospacific PN A34440) according to the Dynal standard protocol, and were incubated with sample for 15 minutes before the sample was flowed over respective sensors.

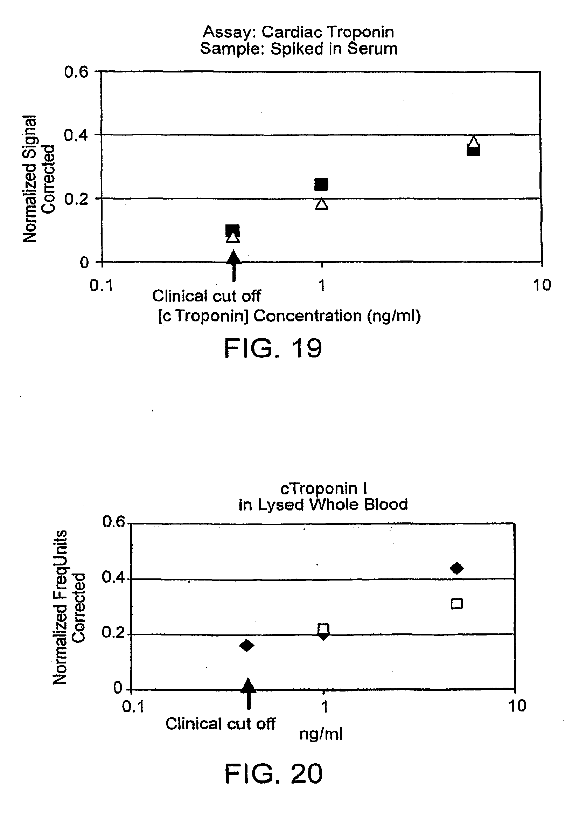

Sensors where functionalized with neutravidin and biotinylated polyclonal antibody (Biospacific PN G-129-C) as described above. The beads/complexes mixture was flowed over the sensor and sensors were washed by flowing buffer at 15 uL/min for 5 mins. Data was observed at a time point of 2 minutes post magnetic field removal. 0 ng/ml samples were used to subtractive correct signal for background level. As shown in FIG. 19, as little as 0.4 mg/ml of cTroponin was detected.

### EXAMPLE IX cTroponin in lysed whole blood

Samples were constructed with 50% whole blood (Texas Biologicals) 50% phosphate buffered saline 0.05% TWEEN® 20 (polyethylene glycol sorbitan monolaurate). Cardiac troponin I (Biospacific) was added at concentrations 0 ng/ml, 0.4 ng/ml, 1 ng/ml and 5 ng/ml. Beads functionalized with anti-cTroponin antibody (Bioscpacific PN A34440) according to the Dynal standard protocol) were incubated with sample for 15 minutes before the results flowed over respective sensors.

Sensors where functionalized with neutravidin and biotinylated anti-cTroponin polyclonal antibody (Biospacific PN G-129-C) as described above. After exposing the sensor to the beads/complexes, sensors were washed by flowing buffer from 50 uL/min through 150 uL/min in increments of 10 ul/min every 30 seconds. Signal data was integrated over a substantial portion of the washing period. 0 ng/ml samples were used to subtractive correct signal for background level. As shown in FIG. 20, as little as 0.4 mg/ml of cTroponin was detected.

### EXAMPLE X CHO-HCP detection

Functionalizing Beads - Dynal M280 tosyl activated beads (described above) were coupled with anti-CHO-TCP polyclonal antibody (CygnusTechnologies F015) using the manufacturer's standard protocol.

Sensors were prepared using the same polyclonal antibody. The polyclonal antibody was biotinylated as described above and applied to FPW sensor surfaces first using a sub layer of biotin PEG disulphide, Polypure PN# 41151-0895 applied to gold FPW surfaces, the Polypure coated subsurface subsequently layered with Neutravidin, Pierce PN 31000, and finally functionalized with the biotinylated capture antibody, in similar fashion as previously described above.

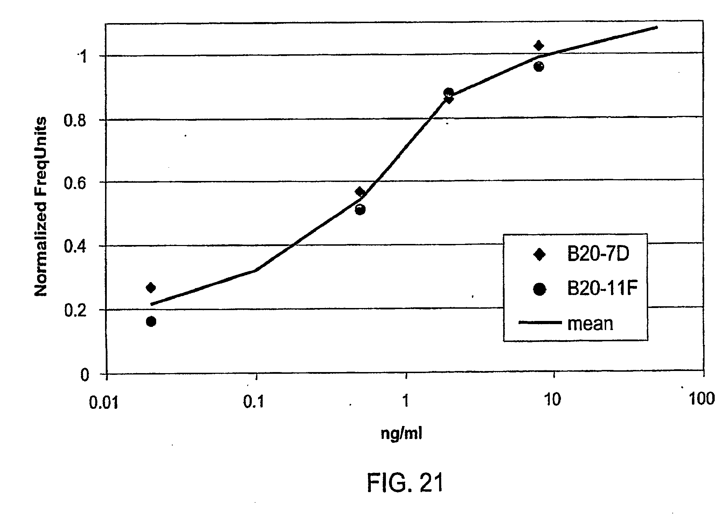

Antigen was diluted in 1X PBS 1% BSA in the concentrations 0 ng/ml, 0.6 ng/ml, 2 ng/ml and 10 ng/ml to make spiked samples. The spiked samples were incubated with functionalized beads at a concentration of about 1x10⁴-5x10⁴ beads/ml. Samples were incubated with the functionalized beads for 2 hrs. A ∼20 minute FPW detection process was performed in similar fashion to the PSA example described earlier. Microparticle loading onto the sensors, post sample incubation, took approximately 3 minutes at 70 ul/min flow rate (210 uL of sample used per sensor). After loading, buffer was introduced, 1x Phosphate Buffered Saline with 0.5% TWEEN® 20 (polyethylene glycol sorbitan monolaurate), yielding a baseline measure of accumulated microparticles. The attractive magnetic field was then removed and microparticles where exposed to washing flow rates in a similar range as in the PSA assay example above. As shown in FIG. 21, methods provided herein are at least as sensitive as standard ELISA assays at detecting host cell protein. Furthermore, the total time for testing CHO-HCP was in the range of 2.5 hrs, as compared to the recommended ELISA 5 hr protocol.

### EXAMPLE XI: Protein A/G Functionalized Sensor

Sensors were functionalized using using protein A/G. FPW chips with gold surfaces were functionalized with protein A/G, by first cleaning the surface of the chip by dipping the chip into acetone for 10 minutes, followed by ethanol for 10 minutes. The chip was then ashed in O₂ plasma (50W, 2 minutes) on both the electrode side and the well side. Five mg of recombinant protein A/G (Pierce PN 21186) was suspended in 2 mls of PBS to give 2.5mg/ml stock solution. Prior to incubation on the gold surfaces, the protein A/G stock solution was diluted to 750 ug/ml. Protein A/G (750 ug/ml) solution was added to the chip in individual wells. The chip was placed in a humidity chamber and incubated for 18 hours. The chip was then throuroughly rinsed with with 1X PBS or DI water, dried and packaged into a cartridge.

The surface can be calibrated, with known concentrations of antibody. In addition, the antibody can be stripped from the calibrated surface and the calibrated surface resused. The antibody can be stripped from the sensor surface by washing the surface with 0.1M phosphoric acid. The stripped surface can be resused more than 10 times.

To load samples, buffer (1 x Phosphate Buffered Saline, 0.05% TWEEN® 20 (polyethylene glycol sorbitan monolaurate)) was added to the cartridge to wet the surface of the sensor. Samples having a final monoclonal antibody concentration of 0.1 mg/ml or 0.01 mg/ml in BSA buffer (prepared by diluting 6 mg/ml of stock solution in the sensor wetting buffer (1 x Phosphate Buffered Saline, 0.05% TWEEN® 20 (polyethylene glycol sorbitan monolaurate)) were introduced into the cartridge and direct frequency shift signals of monoclonal antibody binding to the protein A/G was detected. Detection occurred over 1-4 minutes of sample loading. After 2 minutes of sample loading at 50uL/min (so net 100 uL of diluted sample used) the sample was switched back to buffer and the signal from the sensor leveled out. After each reading and after buffer has flushed out sensor wells, a solution of o.1 M phosphoric acid, was flowed over the sensor for 1 minute, removing (also referred to herein as stripping) the bound monoclonal antibodies and reviving the surface for the next run. The process was repeated three times with the samples. The results for both the 0.1 mg/ml and 0.01 mg/ml samples were reproducible within 5-10%.

## Claims

1. An apparatus for detecting a substance in a fluid, comprising:
a) a fluid chamber having at least one opening for a fluid to enter;
b) a sensor device defining at least a portion of at least one interior surface of the fluid chamber coated with one or more first capture agents capable of binding to one or more substances in the fluid, the sensor device defining an acoustic interaction region of less than 30 microns that acoustically interacts with the fluid in the fluid chamber;
c) a monitoring device to monitor at least one signal output by the sensor device; and
d) a plurality of particles coated with a plurality of second capture agents capable of binding to the one or more substances in the fluid, wherein at least some of the plurality of particles are capable of binding to a surface of the sensor device within the acoustic interaction range, wherein the particle concentration is from 1 x 10² to 1 x 10⁷ particles/ml.

2. The apparatus of claim 1, wherein the particle concentration is from 5 x 10³ to 5 x 10⁵ particles/ml.

3. The apparatus of claim 1, wherein the acoustic interaction region comprises a portion of the sensor device and particles bound at the surface of the sensor device.

4. The apparatus of claim 1, wherein the sensor device is a flexural plate wave device.

5. The apparatus of claim 1, wherein at least some of the plurality of particles bind to the surface of the sensor device within the acoustic interaction region.

6. The apparatus of claim 1, wherein binding of the at least some of the plurality of particles at the surface of the sensor device alters the at least one signal output by the sensor device.

7. The apparatus of claim 1, wherein the plurality of particles are magnetic particles, wherein the apparatus preferably further comprises a source of magnetic flux positioned external to the fluid chamber close to the at least one interior surface of the fluid chamber to create a magnetic field gradient to selectively direct magnetic particles toward the surface of the sensor device.

8. The apparatus of claim 1, wherein the one or more first capture agents are indirectly or directly bound to the surface of the sensor device.

9. The apparatus of claim 1, wherein the one or more first capture agents or the plurality of second capture agents comprise a plurality of polypeptides or comprise an antibody.

10. The apparatus of claim 1, wherein the one or more substances comprise an analyte.

11. The apparatus of claim 1, wherein the one or more substances comprise a tag, wherein the tag preferably is bound by an analyte or is linked to an analyte.

12. The apparatus of claim 1, wherein the one or more substances comprise a binding partner for an analyte.

13. The apparatus of claim 1, wherein the monitoring device monitors (1) at a plurality of points in time; or (2) a change in the at least one signal output.

14. The apparatus of claim 1 further comprising a fluid control device for flowing one or more
fluids over the surface of the sensor device, wherein the one or more fluids preferably comprise a wash flow to remove particles not bound at the surface of the sensor device.

15. The apparatus of claim 1, wherein the acoustic interaction region is between 10 microns and 30 microns or wherein the acoustic interaction range is less than 10 microns.

## Patentansprüche

1. Eine Vorrichtung zur Detektion einer Substanz in einer Flüssigkeit, umfassend:
a) eine Flüssigkeitskammer, die mindestens eine Öffnung hat, damit eine Flüssigkeit hineingelangen kann;
b) eine Sensorvorrichtung, die mindestens einen Teil von mindestens einer Innenfläche der Flüssigkeitskammer definiert und mit einem oder mehreren ersten Einfangagenzien, die zur Bindung an eine oder mehrere Substanzen in der Flüssigkeit in der Lage sind, beschichtet ist, wobei die Sensorvorrichtung einen akustischen Interaktionsbereich von weniger als 30 Mikrometern definiert, der akustisch mit der Flüssigkeit in der Flüssigkeitskammer wechselwirkt;
c) eine Überwachungsvorrichtung, um mindestens ein Ausgangssignal der Sensorvorrichtung zu überwachen, und
d) eine Vielzahl von Partikeln, die mit einer Vielzahl von zweiten Einfangagenzien beschichtet sind, die in der Lage sind, an die eine oder mehreren Substanzen in der Flüssigkeit zu binden, wobei mindestens einige der Vielzahl von Partikeln in der Lage sind, an eine Oberfläche der Sensorvorrichtung innerhalb der akustischen Interaktionsreichweite zu binden, wobei die Partikelkonzentration zwischen 1 x 10² und 1 x 10⁷ Partikel /ml liegt.

2. Die Vorrichtung nach Anspruch 1, wobei die Partikelkonzentration zwischen 5 x 10³ und 5 x 10⁵ Partikel /ml liegt.

3. Die Vorrichtung nach Anspruch 1, wobei der akustische Interaktionsbereich einen Teil der Sensorvorrichtung und Partikel, die an die Oberfläche der Sensorvorrichtung gebunden sind, umfasst.

4. Die Vorrichtung nach Anspruch 1, wobei die Sensorvorrichtung eine Biegeblech-Wellen-Vorrichtung ist.

5. Die Vorrichtung nach Anspruch 1, wobei mindestens einige der Vielzahl von Partikeln an die Oberfläche der Sensorvorrichtung innerhalb des akustischen Interaktionsbereichs binden.

6. Die Vorrichtung nach Anspruch 1, wobei das Binden von mindestens einigen der Vielzahl von Partikeln an die Oberfläche der Sensorvorrichtung das mindestens ein Ausgangssignal der Sensorvorrichtung verändert.

7. Die Vorrichtung nach Anspruch 1, wobei die Vielzahl von Partikeln magnetische Partikel sind, wobei die Vorrichtung vorzugsweise ferner eine Magnetflussquelle umfasst, die außerhalb der Flüssigkeitskammer in der Nähe der mindestens einen Innenfläche der Flüssigkeitskammer positioniert ist, um einen Magnetfeldgradienten zu erzeugen, der selektiv die magnetischen Partikel in Richtung der Oberfläche der Sensorvorrichtung leitet.

8. Die Vorrichtung nach Anspruch 1, wobei das eine oder die mehreren ersten Einfangagenzien indirekt oder direkt an die Oberfläche der Sensorvorrichtung gebunden sind.

9. Die Vorrichtung nach Anspruch 1, wobei das eine oder die mehreren ersten Einfangagenzien oder die Vielzahl von zweiten Einfangagenzien eine Vielzahl von Polypeptiden oder einen Antikörper umfassen.

10. Die Vorrichtung nach Anspruch 1, wobei die eine oder mehrere Substanzen einen Analyten umfassen.

11. Die Vorrichtung nach Anspruch 1, wobei die eine oder mehrere Substanzen einen Marker umfassen, wobei der Marker vorzugsweise von einem Analyten gebunden wird oder an einen Analyten gebunden ist.

12. Die Vorrichtung nach Anspruch 1, wobei die eine oder mehrere Substanzen einen Bindungspartner für einen Analyten umfassen.

13. Die Vorrichtung nach Anspruch 1, wobei die Überwachungsvorrichtung (1) eine Vielzahl von Zeitpunkten, oder (2) eine Änderung des mindestens einen Ausgangssignals überwacht.

14. Die Vorrichtung nach Anspruch 1, ferner umfassend eine Flüssigkeitssteuervorrichtung um eine oder mehrere Flüssigkeiten über die Oberfläche der Sensorvorrichtung strömen zu lassen, wobei die eine oder mehrere Flüssigkeiten vorzugsweise einen Waschstrom umfassen, um Partikel, die nicht an die Oberfläche der Sensorvorrichtung gebunden sind, zu entfernen.

15. Die Vorrichtung nach Anspruch 1, wobei der akustische Interaktionsbereich zwischen 10 Mikrometer und 30 Mikrometer liegt oder wobei die akustische Interaktionsreichweite weniger als 10 Mikron beträgt.

## Revendications

1. Appareil pour détecter une substance dans un fluide, comprenant :
a) une chambre à fluide ayant au moins une ouverture pour permettre l'entrée d'un fluide ;
b) un dispositif de détection définissant au moins une partie d'au moins une surface intérieure de la chambre à fluide traitée avec un ou plusieurs premiers agents de capture capables de se lier à une ou plusieurs substances dans le fluide, le dispositif de détection définissant une région d'interaction acoustique de moins de 30 microns qui interagit acoustiquement avec le fluide dans la chambre à fluide ;
c) un dispositif de surveillance pour contrôler au moins une sortie de signal par le dispositif de détection ; et
d) une pluralité de particules recouvertes d'une pluralité de deuxièmes agents de capture capables de se lier aux une ou plusieurs substances dans le fluide, au moins une partie de la pluralité de particules étant capables de se lier à une surface du dispositif de détection dans la plage d'interaction acoustique, la concentration de particules étant comprise entre 1 x 10² et 1 x 10⁷ particules/ml.

2. Appareil de la revendication 1, dans lequel la concentration de particules est comprise entre 5 x 10³ et 5 x 10⁵ particules/ml.

3. Appareil de la revendication 1, dans lequel la région d'interaction acoustique comprend une partie du dispositif de détection et des particules liées à la surface du dispositif de détection.

4. Appareil de la revendication 1, dans lequel le dispositif de détection est un dispositif à onde de plaque de flexion.

5. Appareil de la revendication 1, dans lequel au moins une partie de la pluralité de particules se lient à la surface du dispositif de détection dans la région d'interaction acoustique.

6. Appareil de la revendication 1, dans lequel la fixation de l'au moins une partie de la pluralité de particules à la surface du dispositif de détection modifie l'au moins une sortie de signal du dispositif de détection.

7. Appareil de la revendication 1, dans lequel la pluralité de particules sont des particules magnétiques, l'appareil comprenant en outre de préférence une source de flux magnétique positionnée à l'extérieur de la chambre à fluide à proximité de l'au moins une surface intérieure de la chambre à fluide pour créer un gradient de champ magnétique pour orienter sélectivement les particules vers la surface du dispositif de détection.

8. Appareil de la revendication 1, dans lequel les un ou plusieurs premiers agents de capture sont indirectement ou directement liés à la surface du dispositif de détection.

9. Appareil de la revendication 1, dans lequel les un ou plusieurs premiers agents de capture ou la pluralité de deuxièmes agents de capture comprennent une pluralité de polypeptides ou comprennent un anticorps.

10. Appareil de la revendication 1, dans lequel les une ou plusieurs substances comprennent un analyte.

11. Appareil de la revendication 1, dans lequel les une ou plusieurs substances comprennent une étiquette, l'étiquette étant de préférence liée par un analyte ou étant liée à un analyte.

12. Appareil de la revendication 1, dans lequel les une ou plusieurs substances comprennent un partenaire de liaison pour un analyte.

13. Appareil de la revendication 1, dans lequel le dispositif de surveillance contrôle (1) à une pluralité de temps ; ou (2) un changement de l'au moins une sortie de signal.

14. Appareil de la revendication 1 comprenant en outre un dispositif de commande de fluide pour faire circuler un ou plusieurs fluides sur la surface du dispositif de détection, dans lequel les un ou plusieurs fluides comprennent de préférence un flux de lavage pour éliminer les particules non liées à la surface du dispositif de détection.

15. Appareil de la revendication 1, dans lequel la région d'interaction acoustique est comprise entre 10 microns et 30 microns ou dans lequel la plage d'interaction acoustique est en-dessous de 10 microns.
